# EUROPEAN PATENT APPLICATION

(11) **EP 4 209 508 A1**
(43) Date of publication of application: **12.07.2023**
(21) Application number: 22305021.2
(22) Date of filing: 11.01.2022
(51) Int. Cl.: C07K 16/18, C07K 16/40, C12N 9/10

(54) **NANOBODIES FOR THE DENEDDYLATING ENZYME NEDP1**

(71) Applicant: Centre national de la recherche scientifique, 75016 Paris (FR); Université de Montpellier, 34090 Montpellier (FR); Vrije Universiteit Brussel, 1050 Brussel (BE)
(72) Inventor: XIRODIMAS, Dimitris, 34730 PRADES-LE-LEZ (FR); ABIDI-AZZOUZ, Naima, 34990 JUVIGNAC (FR); MUYLDERMANS, Serge, 1560 HOEILAART (BE)
(74) Representative: Lavoix

(57) **Abstract**

The present invention relates to isolated single domain antibodies having specificity for deNEDDylating enzyme NEDP1. It concerns in particular to a kit for inhibiting the deNEDDylating enzyme NEDP1, more particularly for the prevention and/or treatment of neurodegenerative diseases such as Amyotrophic Lateral Sclerosis and/or Frontotemporal Degeneration, said kit comprising at least one isolated single domain antibody specific for the deNEDDylating enzyme NEDP1. It further relates to said isolated single domain antibodies for use as inhibitors of the deNEDDylating enzyme NEDP1, in particular for the prevention and/or treatment of neurodegenerative diseases such as ALS/FTD.

## Description

The present invention relates to isolated single domain antibodies having specificity for deNEDDylating enzyme NEDP1.

It concerns in particular a kit for inhibiting the deNEDDylating enzyme NEDP1, more particularly for the prevention and/or treatment of neurodegenerative diseases such as Amyotrophic Lateral Sclerosis and/or Frontotemporal Degeneration, said kit comprising at least one isolated single domain antibody specific for the deNEDDylating enzyme NEDP1.

It further relates to said isolated single domain antibodies for use as inhibitors of the deNEDDylating enzyme NEDP1, in particular for the prevention and/or treatment of neurodegenerative diseases such as ALS/FTD.

Modification of proteins with the ubiquitin-like molecule NEDD8 is essential almost in all tested organisms. Similarly to the modification with ubiquitin, protein NEDDylation depends on the activity of the so-called E1, E2 and E3 enzymes (Abidi, N. & Xirodimas, D. P. Regulation of cancer-related pathways by protein NEDDylation and strategies for the use of NEDD8 inhibitors in the clinic. Endocr. Relat. Cancer 22, T55-T70 (2015); Enchev, R. I., Schulman, B. A. & Peter, M. Protein neddylation: beyond cullin-RING ligases. Nat. Rev. Mol. Cell Biol. 16, 30-44 (2014)). Prior to its activation, NEDD8 has to be processed to expose the C-terminal diGlycine motif. While several enzymes with dual activity towards ubiquitin and NEDD8 have been reported as NEDD8 processing enzymes, the NEDP1 (DEN1, SENP8) cysteine protease has specific NEDD8 processing activity (Abidi, N. & Xirodimas, D. P. Regulation of cancer-related pathways by protein NEDDylation and strategies for the use of NEDD8 inhibitors in the clinic. Endocr. Relat. Cancer 22, T55-T70 (2015); Enchev, R. I., Schulman, B. A. & Peter, M. Protein neddylation: beyond cullin-RING ligases. Nat. Rev. Mol. Cell Biol. 16, 30-44 (2014)). Upon its processing, NEDD8 is activated by a single E1 enzyme (heterodimer Uba3/APPBP1) and conjugated through the activity of two E2 conjugating enzymes (Ube2M, Ube2F) and multiple E3-ligases (Abidi, N. & Xirodimas, D. P. Regulation of cancer-related pathways by protein NEDDylation and strategies for the use of NEDD8 inhibitors in the clinic. Endocr. Relat. Cancer 22, T55-T70 (2015); Enchev, R. I., Schulman, B. A. & Peter, M. Protein neddylation: beyond cullin-RING ligases. Nat. Rev. Mol. Cell Biol. 16, 30-44 (2014)). The activation and conjugation of NEDD8 by the above-described specific NEDD8 enzymes defines the so-called canonical pathway. However, under conditions of proteotoxic stress, in addition to the conjugation of NEDD8 by the canonical pathway, NEDD8 is activated and conjugated by enzymes of the ubiquitin system, leading to the formation of hybrid ubiquitin-NEDD8 chains (Leidecker, O., Matic, I., Mahata, B., Pion, E. & Xirodimas, D. P. The ubiquitin E1 enzyme Ube1 mediates NEDD8 activation under diverse stress conditions. Cell Cycle Georget. Tex 11, 1142-1150 (2012); Hjerpe, R. et al. Changes in the ratio of free NEDD8 to ubiquitin triggers NEDDylation by ubiquitin enzymes. Biochem. J. 441, 927-936 (2012)). This defines the so-called atypical pathway that is involved in the formation of nuclear protein aggregates and control of the nuclear Ubiquitin Proteasome System (Maghames, C. M. et al. NEDDylation promotes nuclear protein aggregation and protects the Ubiquitin Proteasome System upon proteotoxic stress. Nat. Commun. 9, (2018); Li, J. et al. NEDD8 Ultimate Buster 1 Long (NUB1L) Protein Suppresses Atypical Neddylation and Promotes the Proteasomal Degradation of Misfolded Proteins. J. Biol. Chem. 290, 23850-23862 (2015)).

The action of NEDD8 conjugating enzymes is finely balanced by the activity of deconjugating (deNEDDylating) enzymes. Similarly to NEDD8 processing, several proteases with dual activity towards ubiquitin and NEDD8 can promote NEDD8 deconjugation from substrates (Abidi, N. & Xirodimas, D. P. Regulation of cancer-related pathways by protein NEDDylation and strategies for the use of NEDD8 inhibitors in the clinic. Endocr. Relat. Cancer 22, T55-T70 (2015); Enchev, R. I., Schulman, B. A. & Peter, M. Protein neddylation: beyond cullin-RING ligases. Nat. Rev. Mol. Cell Biol. 16, 30-44 (2014)). However, the two specific deNEDDylating enzymes are the COP9 signalosome and NEDP1. COP9, a zinc metalloprotease, has minimal affinity towards NEDD8 but specifically deconjugates NEDD8 from cullins, the best established target for NEDD8 (Abidi, N. & Xirodimas, D. P. Regulation of cancer-related pathways by protein NEDDylation and strategies for the use of NEDD8 inhibitors in the clinic. Endocr. Relat. Cancer 22, T55-T70 (2015); Enchev, R. I., Schulman, B. A. & Peter, M. Protein neddylation: beyond cullin-RING ligases. Nat. Rev. Mol. Cell Biol. 16, 30-44 (2014)). NEDP1 on the other hand displays deNEDDylating activity mainly towards non-cullin substrates (Aoki, I., Higuchi, M. & Gotoh, Y. NEDDylation controls the target specificity of E2F1 and apoptosis induction. Oncogene 32, 3954-3964 (2013); Vogl, A. M. et al. Neddylation inhibition impairs spine development, destabilizes synapses and deteriorates cognition. Nat. Neurosci. 18, 239-251 (2015); Broemer, M. et al. Systematic in vivo RNAi analysis identifies IAPs as NEDD8-E3 ligases. Mol. Cell 40, 810-822 (2010); Christmann, M. et al. Control of Multicellular Development by the Physically Interacting Deneddylases DEN1/DenA and COP9 Signalosome. PLoS Genet. 9, e1003275 (2013); Coleman, K. E. et al. SENP8 limits aberrant neddylation of NEDD8 pathway components to promote cullin-RING ubiquitin ligase function. eLife 6, (2017); Mergner, J., Heinzlmeir, S., Kuster, B. & Schwechheimer, C. DENEDDYLASE1 Deconjugates NEDD8 from Non-Cullin Protein Substrates in Arabidopsis thaliana. Plant Cell 27, 741-753 (2015); Watson, I. R. et al. Chemotherapy induces NEDP1-mediated destabilization of MDM2. Oncogene 29, 297-304 (2010); Xirodimas, D. P. et al. Ribosomal proteins are targets for the NEDD8 pathway. EMBO Rep. 9, 280-286 (2008); Li, T., Guan, J., Huang, Z., Hu, X. & Zheng, X. RNF168-mediated H2A neddylation antagonizes ubiquitylation of H2A and regulates DNA damage repair. J. Cell Sci. 127, 2238-2248 (2014); Ma, T. et al. RNF111-Dependent Neddylation Activates DNA Damage-Induced Ubiquitination. Mol. Cell 49, 897-907 (2013); Guan, J., Yu, S. & Zheng, X. NEDDylation antagonizes ubiquitination of proliferating cell nuclear antigen and regulates the recruitment of polymerase η in response to oxidative DNA damage. Protein Cell (2017) doi:10.1007/s13238-017-0455-x.; EI Motiam, A. et al. Interplay between SUMOylation and NEDDylation regulates RPL11 localization and function. FASEB J. 33, 643-651 (2019)) and its deletion causes a dramatic increase in global protein NEDDylation (Coleman, K. E. et al. SENP8 limits aberrant neddylation of NEDD8 pathway components to promote cullin-RING ubiquitin ligase function. eLife 6, (2017); Mergner, J., Heinzlmeir, S., Kuster, B. & Schwechheimer, C. DENEDDYLASE1 Deconjugates NEDD8 from Non-Cullin Protein Substrates in Arabidopsis thaliana. Plant Cell 27, 741-753 (2015); Bailly, A. P. et al. The Balance between Mono- and NEDD8-Chains Controlled by NEDP1 upon DNA Damage Is a Regulatory Module of the HSP70 ATPase Activity. Cell Rep. 29, 212-224.e8 (2019); Keuss, M. J. et al. Unanchored tri-NEDD8 inhibits PARP-1 to protect from oxidative stress-induced cell death. EMBO J. e100024 (2019) doi:10.15252/embj.2018100024) with the accumulation of poly-NEDD8 chains through lysines K11/K48 as a prominent effect (Coleman, K. E. et al. SENP8 limits aberrant neddylation of NEDD8 pathway components to promote cullin-RING ubiquitin ligase function. eLife 6, (2017); Bailly, A. P. et al. The Balance between Mono- and NEDD8-Chains Controlled by NEDP1 upon DNA Damage Is a Regulatory Module of the HSP70 ATPase Activity. Cell Rep. 29, 212-224.e8 (2019)). Importantly, both deNEDDylating activities control the canonical NEDD8 pathway (Abidi, N. & Xirodimas, D. P. Regulation of cancer-related pathways by protein NEDDylation and strategies for the use of NEDD8 inhibitors in the clinic. Endocr. Relat. Cancer 22, T55-T70 (2015); Enchev, R. I., Schulman, B. A. & Peter, M. Protein neddylation: beyond cullin-RING ligases. Nat. Rev. Mol. Cell Biol. 16, 30-44 (2014); Bailly, A. P. et al. The Balance between Mono- and NEDD8-Chains Controlled by NEDP1 upon DNA Damage Is a Regulatory Module of the HSP70 ATPase Activity. Cell Rep. 29, 212-224.e8 (2019)). While the function of the COP9 signalosome as a key component of the NEDD8 pathway through regulation of the Cullin Ring Ligases has been established, the role of NEDP1 in homeostatic and stress responses is only now emerging (Abidi, N. & Xirodimas, D. P. Regulation of cancer-related pathways by protein NEDDylation and strategies for the use of NEDD8 inhibitors in the clinic. Endocr. Relat. Cancer 22, T55-T70 (2015); Enchev, R. I., Schulman, B. A. & Peter, M. Protein neddylation: beyond cullin-RING ligases. Nat. Rev. Mol. Cell Biol. 16, 30-44 (2014)). Recent studies indicated a role for NEDP1 in tumour progression; The upregulation of NEDD8 in hepatocellular carcinoma was related to a decrease in the levels of NEDP1. Importantly, decrease in NEDP1 levels provides resistance of tumour cells to DNA damage induced apoptosis (Broemer, M. et al. Systematic in vivo RNAi analysis identifies IAPs as NEDD8-E3 ligases. Mol. Cell 40, 810-822 (2010); Bailly, A. P. et al. The Balance between Mono- and NEDD8-Chains Controlled by NEDP1 upon DNA Damage Is a Regulatory Module of the HSP70 ATPase Activity. Cell Rep. 29, 212-224.e8 (2019); Delgado, T. C. et al. Neddylation, a novel paradigm in liver cancer. Transl. Gastroenterol. Hepatol. 3, 37-37 (2018)).

Targeting the NEDD8 pathway is a promising therapeutic approach. Inhibitors for the NEDD8 E1 enzyme (MLN4924/Pevonedistat) are in Phase III clinical trials for cancer Treatment (Abidi, N. & Xirodimas, D. P. Regulation of cancer-related pathways by protein NEDDylation and strategies for the use of NEDD8 inhibitors in the clinic. Endocr. Relat. Cancer 22, T55-T70 (2015); Ying, J., Zhang, M., Qiu, X. & Lu, Y. Targeting the neddylation pathway in cells as a potential therapeutic approach for diseases. Cancer Chemother. Pharmacol. 81, 797- 808 (2018); Soucy, T. A., Dick, L. R., Smith, P. G., Milhollen, M. A. & Brownell, J. E. The NEDD8 Conjugation Pathway and Its Relevance in Cancer Biology and Therapy. Genes Cancer 1, 708-716 (2010)).The promising anti-tumour effects of MLN4924 have generated an interest in the field and additional components of the NEDD8 pathway including E2 conjugating enzymes, E3-ligases and the COP9 signalosome have been targeted (Abidi, N. & Xirodimas, D. P. Regulation of cancer-related pathways by protein NEDDylation and strategies for the use of NEDD8 inhibitors in the clinic. Endocr. Relat. Cancer 22, T55-T70 (2015); Schlierf, A. et al. Targeted inhibition of the COP9 signalosome for treatment ofcancer. Nat. Commun. 7, (2016); Zhou, H. et al. High-Affinity Peptidomimetic Inhibitors of the DCN1-UBC12 Protein-Protein Interaction. J. Med. Chem. 61, 1934-1950 (2018)).

Apart for their potential clinical testing and therapeutic use, such compounds provide invaluable tools to study the function of the target in question. However, specific inhibitors of the deNEDDylating enzyme NEDP1 do not currently exist.

Nanobodies (Nbs) are the smallest antigen binding fragments, only 15 kDa, containing the full antigen-binding capacity of the original heavy chain. In addition to the benefits of conventional antibodies, such as high affinity and selectivity for a target, easy tailoring into pluripotent constructs and low inherent toxicity, Nbs have technological and biophysical advantages that outperform conventional antibodies. Nbs are only a tenth of the size of a conventional antibody able to penetrate tissues more effectively than conventional antibodies and they can recognize uncommon or hidden epitopes (De Genst, E. et al. Molecular basis for the preferential cleft recognition by dromedary heavy-chain antibodies. Proc. Natl. Acad. Sci. U. S. A. 103, 4586-4591 (2006); Harmsen, M. M. & De Haard, H. J. Properties, production, and applications of camelid single-domain antibody fragments. Appl. Microbiol. Biotechnol. 77, 13-22 (2007)). They are naturally soluble in aqueous solution and do not have a tendency to aggregate (Muyldermans, S. Single domain camel antibodies: current status. J. Biotechnol. 74, 277-302 (2001)). They can be easily expressed in cells as fusion proteins enabling the recognition of the target of interest in living cells. These features have already lead to a number of biotechnological and medical applications in which Nbs excel other antibody formats (Jovčevska, I. & Muyldermans, S. The Therapeutic Potential of Nanobodies. BioDrugs (2019) doi:10.1007/s40259-019-00392-z.; Revets, H., De Baetselier, P. & Muyldermans, S. Nanobodies as novel agents for cancer therapy. Expert Opin. Biol. Ther. 5, 111-124 (2005)).

Here, the inventors have developed and characterized Nbs that specifically inhibit NEDP1 activity, which currently represent the first-in-class inhibitors for NEDP1.

The present invention thus relates to nanobodies or single domain antibodies that are inhibitors of the deNEDDylating enzyme NEDP1 and can be used for the prevention and/or treatment of neurodegenerative diseases such as ALS/FTD.

The inventors of the present invention have indeed discovered new isolated single domain antibodies specific for the deNEDDylating enzyme NEDP1 as mentioned in the Tables 1 to 3 below.

**Table 1**

| **Name of the Ab** | **CDR1 amino acid sequence** | **CDR2 amino acid sequence** | **CDR3 amino acid sequence** |
|---|---|---|---|
| R2DP44 | GSILRINV (SEQ ID NO:1) | ITSGGST (SEQ ID NO:2) | AAAGGS (SEQ ID NO:3) |
| R2DP58 | GSIRSINV (SEQ ID NO:4) | ITSGGYT (SEQ ID NO:5) | KGVAVP (SEQ ID NO:6) |
| R2DP61 | KSIFSINV (SEQ ID NO:7) | VTSGGST (SEQ ID NO:9) | AAVERGMDY (SEQ ID NO:9) |
| R2DP80 | GSIFKINV (SEQ 10 NO:10) | ITSGGST (SEQ ID NO:11) | ATPGSV (SEQ ID NO:12) |
| R2DP16 | GSDFSINV (SEQ ID NO:13) | ISGGGAT (SEQ ID NO:14) | RGIVAGADY (SEQ ID NO:15) |
| R2DP23 | GSIASINV (SEQ ID NO:16) | VANGGQS (SEQ ID NO:17) | IDLEGLRIGN (SEQ ID NO:18) |
| R2DP47 | GSISSINV (SEQ 10 NO:19) | ISSFGIT (SEQ ID NO:20) | KGPNTD (SEQ ID NO:21) |
| R2DP49 | GIIDSINV ((SEQ ID NO:22) | ITSRGIT (SEQ ID NO:23) | TYLGRTS (SEQ ID NO:24) |
| R2DP69 | GSIFHINL (SEQ ID NO:25) | ITGGGST (SEQ ID NO:26) | RGLVPGKDDY (SEQ ID NO:27) |
| R2DP19 | GSIFSQNV (SEQ ID NO:28) | ITSGGNT (SEQ ID NO:29) | AGSSWVNDY (SEQ ID NO:30) |
| R2DP93 | GSIFSINV (SEQ ID NO:31) | IINAGTT (SEQ ID NO:32) | AAYLSGRTV (SEQ ID NO:33) |
| R2DP11 | GNIFNINV (SEQ ID NO:34) | ITSGRLT (SEQ ID NO:35) | AAARPGGYY (SEQ ID NO:36) |
| R2DP46 | GSPFSIGT (SEQ ID NO:37) | ITRGGAT (SEQ ID NO:38) | SVWAGDRAY (SEQ ID NO:39) |
| R2DP67 | GSPFSIGT (SEQ ID NO:40) | ITRGGAT (SEQ ID NO:41) | SVWAGDRAF (SEQ ID NO:42) |
| R2DP34 | GSATRINV (SEQ ID NO:43) | VTSGGST (SEQ ID NO:44) | AAVEDEYVY (SEQ ID NO:45) |
| R2DP78 | GITFTLNV (SEQ ID NO:46) | ISNGAIS (SEQ ID NO:47) | A TVIGGSRF (SEQ ID NO:48) |
| R2DP12 | GSTSSFNV (SEQ ID NO:49) | ITSGGIT (SEQ ID NO:50) | GGVVDGRVY (SEQ ID NO:51) |
| R2DP57 | GTTSSFNV (SEQ ID NO:52) | ITSGGST (SEQ ID NO:53) | GGVVGGRVY (SEQ ID NO:54) |
| R2DP62 | GSIVSSNA (SEQ ID NO:55) | IANSGIT (SEQ ID NO:56) | AAIFNQQFA (SEQ ID NO:57) |
| R2DP35 | GVMFSTGV (SEQ ID NO:58) | ITSGGLT (SEQ ID NO:59) | NVRGY (SEQ ID NO:60) |
| R2DP55 | GFTFDDHA (SEQ ID NO:61) | LSNSDGTT (SEQ ID NO:62) | AICHFRERDGSAWFEGS (SEQ ID NO:63) |

**Table 2**

| **Name of the Ab** | **Full amino acid sequence** |
|---|---|
| R2DP44 | QVQLQESGGGLVQPGGSLRLSCAASGSILRINVMGWYRQASGKQRESVAAITSGGST TYANSVRGRFTISRDNAKNTVYLQMNSLKPEDTAVYYCAAAGGSWGQGTQVTVSS (SEQ ID NO:64) |
| R2DP58 | QVQLQESGGGLVQPGGSLRLSCAASGSIRSINVMGWYRQAPGKQRELVAGITSGGYT NYADSVKGRFTISRDNAKNTVYLQMNSLKPEDTAVYYCKGVAVPWGQGTQVTVSS (SEQ ID NO:65) |
| R2DP61 | QVQLQESGGGLVQAGGSLRLSCAASKSIFSINVMGWYRQAPGKQRESVAAVTSGGS TNYRDSVKGRFTISRDNAKNTVYLQMNRLKPEDTAVYYCAAVERGMDYWGQGTQVT VSS (SEQ ID NO:66) |
| R2DP80 | QVQLQESGGGLVQAGGSLRLSCAASGSIFKINVMAWYRQAPGKQRESVAAITSGGST NYAASVKGRFVISRDNAKNTVYLQMDSLKPEDTAVYYCATPGSVPGQGTQVTVSS (SEQ ID NO:67) |
| R2DP16 | QVQLQESGGGLVQPGGSLRLSCAASGSDFSINVMGWYRQAPGKERESVATISGGGA TAYAYSVKGRFTISRDNAKNTVYLQMNKLKPEDTAVYFCRGIVAGADYWGQGTQVTV SS (SEQ ID NO:68) |
| R2DP23 | QVQLQESGGGLVQPGGSLRLSCAASGSIASINVMAWYRQAPGKQRESVAAVANGGQ STYADSVKGRFTISRDNAKNTMYLQMNSLKPEDTAVYYCIDLEGLRIGNWGQGTQVTV SS (SEQ ID NO:69) |
| R2DP47 | QVQLQESGGGLVEPGGSLRLSCAASGSISSINVMAWYRQAPGKQRESVAAISSFGITT YSETVKGRFTASRDNAKNTMYLQMNNLKPEDTAVYYCKGPNTDWGQGTQVTVSS (SEQ ID NO:70) |
| R2DP49 | QVQLQESGGGLVQPGGSLRLSCAASGIIDSINVMAWYRQAPGKQRESVATITSRGITA YADSVKGRFIISRDNAKNTMYLQMNSLKPEDTAVYYCTYLGRTSWGQGTQVTVSS (SEQ ID NO:71) |
| R2DP69 | QVQLQESGGGLVQPRGSLRLSCAASGSIFHINLMGWYRQAPGKQRESVAVITGGGST TYRDSVKGRFTISRDNAKNTVYLQMNSLKPEDTGVYYCRGLVPGKDDYWGQGTQVT VSS (SEQ ID NO:72) |
| R2DP19 | QVQLQESGGGLVQAGGSLRLSCVVSGSIFSQNVMGWYRQAPGKQRESVAAITSGGN TNYRDSVKGRFTISRDNAKDTVYLQMNRLKPEDTAVYYCAGSSWVNDYWGQGTQVT VSS (SEQ ID NO:73) |
| R2DP93 | QVQLQESGGGLVQAGGSLRLSCAASGSIFSINVMAWYRQAPGKQREAVASIINAGTTI YANSVKGRFTISRDNVKNALYLQMNSLKPEDTAVYYCAAYLSGRTVWGQGTQVTVSS (SEQ ID NO:74) |
| R2DP11 | QVQLQESGGGLVQAGGSLRLSCVASGNIFNINVMAWYRQPPGKQRESVAAITSGRLT TYNASVKGRFTIYRDNAENTVYLQMNGLKPEDTGVYYCAAARPGGYYWGQGTQVTV SS (SEQ ID NO:75) |
| R2DP46 | QVQLQESGGGLVQAGGSLRLSCAASGSPFSIGTMAWYRQAPGNQRASVAAITRGGA TTYANSVKGRFTVSRDNAKNTVYLQMNSLKPEDTAAYYCSVWAGDRAYWGQGTQVT VSS (SEQ ID NO:76) |
| R2DP67 | QVQLQESGGGLVQAGGSLRLSCAASGSPFSIGTMAWYRQAPGNQRASVAAITRGGA TTYANSVKGRFTVSRDNAKNTVYLQMNSLKPEDTAVYYCSVWAGDRAFWGQGTQVT VSS (SEQ ID NO:77) |
| R2DP34 | QVQLQESGGGLVQAGGSLRLSCAASGSATRINVMSWYRQAPGKKRESVAVVTSGGS TNYLASVKGRFTISRDNAKNTVYLQMNSLKPEDTAVYYCAAVEDEYVYWGKGTQVTV SS (SEQ ID NO:78) |
| R2DP78 | QVQLQESGGGLVQAGGSLRLSCAASGITFTLNVMGWYRQAPGKQREAVATISNGAIS NYANSVKGRFTISRDNAKNTVYLQMNSLKPEDTAVYYCATVIGGSRFAGQGTQVTVSS (SEQ ID NO:79) |
| R2DP12 | QVQLQESGGGLVQAGGSLRLSCAASGSTSSFNVMAWYHQAPGKQRESVASITSGGI TVYRDSVKGRFAISSDNAKNTVFLQMNSLKPEDTGVYYCGGVVDGRVYRGQGTQVT VSS (SEQ ID NO:80) |
| R2DP57 | QVQLQESGGGLVQAGGSLRLSCAVSGTTSSFNVMNWHHQAPGKQRESVASITSGGS TLYRDSVKGRLTISRDNAKNAVYLQMNSLKPEDTGVYYCGGVVGGRVYWGQGTQVT VSS (SEQ ID NO:81) |
| R2DP62 | QVQLQESGGGLVQAGGSLRLSCAASGSIVSSNAMSWYRQAPGKQRESVAAIANSGIT TYRDSVKGRFTISRDNTKNTMYLQMNSLRPEDTAVYYCAAIFNQQFAWGQGTQVTVS S (SEQ ID NO:82) |
| R2DP35 | QVQLQESGGGLVQPGGSLTLSCAASGVMFSTGVMGWYRQAPGKQREAVAAITSGGL TTYKDSVKGRFAISRDNTKSTGYLQMNRLRPEDTAVYYCNVRGYWGQGTQVTVSS (SEQ ID NO:83) |
| R2DP55 | QVQLQESGGGLVQAGGSLRVSCATSGFTFDDHAIGWFRQAPGKEREAVSCLSNSDG TTYYTDSVKGRFTASSDNAKKTGYLQMKSLKPEDTAVYYCAICHFRERDGSAWFEGS WGQGTQ VTVSS (SEQ ID NO:84) |

**Table 3**

| **Name of the Ab** | **Full nucleic acid sequence (with CDR1, CDR2 and CDR3 respectively underlined)** |
|---|---|
| R2DP44 | CAGGTGCAGCTGCAGGAGTCTGGGGGAGGCTTGGTGCAGCCTGGGGGGTCTCTG AGACTCTCCTGTGCAGCCTCTGGAAGCATCTTACGTATCAATGTCATGGGCTGGTA CCGCCAGGCTTCAGGGAAGCAGCGGGAGTCGGTCGCAGCTATTACTAGTGGTGG TAGCACAACGTATGCAAACTCCGTGAGGGGCCGATTCACCATCTCCAGAGACAAC GCCAAGAACACGGTGTATCTGCAAATGAACAGCCTGAAACCTGAGGACACAGCCG TCTATTACTGTGCAGCCGCCGGGGGTTCCTGGGGCCAGGGGACCCAGGTCACCG TCTCCAGCGGCCGCTACCCGTACGACGTTCCGGACTACGGTTCCGGCCGAGCAT AG (SEQ ID NO:85) |
| R2DP58 | CAGGTGCAGCTGCAGGAGTCTGGGGGAGGCTTGGTGCAGCCTGGGGGGTCTCTG AGACTCTCCTGTGCAGCCTCTGGAAGCATCCGCAGTATCAATGTAATGGGCTGGT ACCGCCAGGCTCCAGGGAAGCAGCGCGAGTTGGTCGCAGGTATTACTAGTGGTG GTTACACAAACTATGCAGACTCCGTGAAGGGCCGATTCACCATCTCCAGAGACAA CGCCAAGAACACGGTGTATCTGCAAATGAACAGCCTGAAACCTGAGGACACGGCC GTCTATTACTGCAAGGGGGTAGCTGTTCCCTGGGGCCAGGGGACCCAGGTCACC |
| | GTCTCCAGCGGCCGCTACCCGTACGACGTTCCGGACTACGGTTCCGGCCGAGCA TAG (SEQ ID NO:86) |
| R2DP61 | CAGGTGCAGCTGCAGGAGTCTGGGGGAGGCTTGGTGCAGGCTGGGGGGTCTCT GAGACTCTCCTGTGCAGCCTCTAAAAGCATCTTCAGTATCAATGTCATGGGCTGGT ACCGCCAGGCTCCAGGGAAGCAGCGCGAGTCTGTCGCAGCTGTTACTAGTGGTG GAAGCACAAACTATCGAGACTCCGTGAAGGGCCGATTCACCATCTCCAGAGATAA CGCCAAGAACACGGTGTATCTGCAAATGAACCGCCTGAAACCTGAGGACACAGCC GTCTATTACTGTGCAGCAGTTGAAAGGGGGATGGACTACTGGGGCCAGGGGACC CAGGTCACCGTCTCCAGCGGCCGCTACCCGTACGACGTTCCGGACTACGGTTCC GGCCGAGCATAG (SEQ ID NO:87) |
| R2DP80 | CAGGTGCAGCTGCAGGAGTCTGGGGGAGGCTTGGTGCAGGCTGGGGGGTCTCT GAGACTCTCCTGTGCAGCCTCTGGAAGCATCTTCAAGATCAATGTCATGGCCTGGT ACCGCCAGGCTCCAGGGAAGCAGCGCGAGTCGGTCGCAGCTATTACTAGTGGTG GTAGCACAAACTATGCAGCATCCGTGAAGGGCCGATTCGTCATCTCCAGAGACAA CGCCAAGAACACGGTGTATCTGCAAATGGACAGCCTGAAACCTGAGGACACAGCC GTCTATTACTGTGCAACCCCGGGGTCCGTCCCCGGACAGGGGACCCAGGTCACC GTCTCCAGCGGCCGCTACCCGTACGACGTTCCGGACTACGGTTCCGGCCGAGCA TAG (SEQ ID NO:88) |
| R2DP16 | CAGGTGCAGCTGCAGGAGTCTGGGGGAGGCTTGGTGCAGCCTGGGGGGTCTCTG AGACTCTCCTGTGCGGCCTCTGGAAGCGACTTCAGTATCAATGTCATGGGCTGGT ACCGCCAGGCTCCAGGGAAGGAGCGCGAATCGGTCGCAACCATTTCCGGTGGAG GTGCGACTGCCTATGCATACTCCGTGAAGGGCCGATTCACCATTTCCAGAGACAA CGCCAAGAACACGGTGTATCTGCAAATGAACAAACTGAAACCAGAGGACACGGCC GTCTATTTTTGTCGAGGGATAGTAGCTGGTGCAGACTACTGGGGCCAGGGGACCC AGGTCACCGTCTCCAGCGGCCGCTACCCGTACGACGTTCCGGACTACGGTTCCG GCCGAGCATAG (SEQ ID NO:89) |
| R2DP23 | CAGGTGCAGCTGCAGGAGTCTGGGGGAGGCTTGGTGCAGCCTGGAGGGTCTCTG AGACTCTCCTGCGCAGCCTCTGGAAGCATCGCTAGTATCAATGTCATGGCCTGGT ACCGCCAGGCTCCAGGGAAGCAGCGCGAGTCGGTCGCAGCTGTTGCTAATGGTG GTCAATCAACCTATGCAGACTCCGTGAAGGGCCGATTCACCATCTCCAGAGACAA CGCCAAGAACACGATGTATCTGCAAATGAACAGCCTGAAACCTGAGGACACGGCC GTCTATTACTGTATAGATTTAGAAGGACTACGCATTGGGAACTGGGGCCAGGGGA CCCAGGTCACCGTCTCCAGCGGCCGCTACCCGTACGACGTTCCGGACTACGGTT CCGGCCGAGCATAG (SEQ ID NO:90) |
| R2DP47 | CAGGTGCAGCTGCAGGAGTCTGGGGGAGGCTTGGTGGAGCCTGGGGGGTCTCT GAGACTCTCCTGTGCAGCCTCTGGAAGCATCTCCAGTATCAATGTCATGGCCTGG TACCGCCAGGCTCCAGGGAAGCAGCGCGAGTCGGTCGCAGCAATTAGTAGTTTTG GTATCACAACCTACTCAGAGACCGTGAAGGGCCGATTCACCGCCTCCAGAGACAA |
| | CAGGTGCAGCTGCAGGAGTCTGGGGGAGGCTTGGTGCAGCCTGGGGGGTCTCTG |
| R2DP49 | CAGGTGCAGCTGCAGGAGTCTGGGGGAGGCTTGGTGCAGCCTGGGGGGTCTCTG AGACTCTCCTGTGCAGCCTCTGGAATCATCGACAGTATCAATGTCATGGCCTGGTA CCGCCAGGCTCCAGGGAAGCAGCGCGAGTCGGTCGCGACTATTACTAGTAGAGG TATTACTGCCTATGCAGACTCCGTGAAGGGCCGATTCATCATCTCCAGAGACAACG CCAAGAACACGATGTATCTGCAAATGAACAGCCTGAAACCTGAGGACACGGCCGT CTATTACTGCACATATTTAGGCCGGACCAGCTGGGGCCAGGGGACCCAGGTCACC GTCTCCAGCGGCCGCTACCCGTACGACGTTCCGGACTACGGTTCCGGCCGAGCA TAG (SEQ ID NO:92) |
| R2DP69 | CAGGTGCAGCTGCAGGAGTCTGGAGGAGGCTTGGTGCAGCCTAGGGGGTCTCTG AGACTCTCCTGTGCAGCCTCTGGAAGCATCTTCCATATCAATCTCATGGGCTGGTA CCGCCAGGCTCCAGGGAAGCAGCGCGAGTCGGTCGCAGTTATTACTGGTGGTGG TTCTACAACCTATCGAGACTCCGTGAAGGGCCGATTCACCATCTCCAGAGACAAC GCCAAGAACACGGTGTATCTGCAAATGAACAGCCTGAAACCTGAAGACACAGGTG TCTATTACTGTAGAGGATTAGTACCTGGTAAGGATGACTACTGGGGCCAGGGGAC CCAGGTCACCGTCTCCAGCGGCCGCTACCCGTACGACGTTCCGGACTACGGTTC CGGCCGAGCATAG (SEQ ID NO:93) |
| R2DP19 | CAGGTGCAGCTGCAGGAGTCTGGGGGAGGCTTGGTGCAGGCTGGGGGGTCTCT GAGACTCTCCTGTGTAGTATCTGGAAGCATCTTCAGTCAGAATGTTATGGGCTGGT ACCGCCAGGCTCCAGGGAAACAGCGCGAGTCGGTCGCAGCTATTACTAGTGGCG GTAACACAAATTATAGAGACTCCGTGAAGGGCCGATTCACCATCTCCAGAGACAAC GCCAAGGATACGGTGTATCTGCAAATGAACAGACTGAAACCTGAGGACACAGCCG TCTATTACTGTGCAGGAAGTTCCTGGGTTAATGACTACTGGGGCCAGGGGACCCA GGTCACCGTCTCCAGCGGCCGCTACCCGTACGACGTTCCGGACTACGGTTCCGG CCGAGCATAG (SEQ ID NO:94) |
| R2DP93 | CAGGTGCAGCTGCAGGAGTCTGGGGGAGGCTTGGTGCAGGCTGGGGGGTCTCT GAGACTCTCCTGTGCAGCCTCTGGAAGCATCTTCAGTATCAATGTCATGGCGTGGT ACCGCCAGGCTCCAGGGAAGCAGCGCGAGGCGGTCGCATCTATTATTAACGCCG GTACCACAATCTATGCAAACTCCGTGAAGGGCCGATTCACCATCTCCAGAGACAAC GTCAAGAACGCTTTGTATCTGCAAATGAACAGCCTGAAACCGGAGGACACAGCCG TCTATTACTGTGCAGCATATTTAAGTGGACGCACCGTCTGGGGCCAGGGGACCCA GGTCACCGTCTCCAGCGGCCGCTACCCGTACGACGTTCCGGACTACGGTTCCGG CCGAGCATAG (SEQ ID NO:95) |
| R2DP11 | CAGGTGCAGCTGCAGGAGTCTGGGGGAGGCTTGGTGCAGGCTGGGGGGTCTCT GAGACTCTCCTGTGTAGCCTCTGGAAACATCTTCAATATCAATGTCATGGCCTGGT |
| | ACCGCCAGCCTCCAGGGAAGCAGCGCGAGTCGGTCGCAGCTATTACTAGTGGTC GTCTCACAACTTATAACGCCTCCGTAAAGGGCCGATTCACCATCTACAGAGACAAC GCCGAGAACACGGTGTATCTGCAAATGAACGGCCTGAAACCTGAGGACACAGGTG TCTATTACTGCGCAGCAGCCCGGCCGGGGGGATACTACTGGGGTCAGGGGACCC AGGTCACCGTCTCCAGCGGCCGCTACCCGTACGACGTTCCGGACTACGGTTCCG GCCGAGCATAG (SEQ ID NO:96) |
| R2DP46 | CAGGTGCAGCTGCAGGAGTCTGGAGGAGGCTTGGTGCAGGCTGGAGGGTCTCTG AGACTCTCCTGTGCAGCCTCTGGAAGTCCCTTCAGTATCGGTACCATGGCCTGGT ACCGCCAGGCTCCAGGGAATCAGCGCGCATCGGTCGCAGCTATTACTAGGGGTG GTGCGACAACCTATGCAAACTCCGTGAAGGGCCGATTCACCGTCTCTAGAGACAA CGCCAAGAACACAGTGTATCTGCAAATGAACAGCCTGAAACCTGAGGACACGGCC GCCTATTATTGTAGTGTCTGGGCCGGCGATAGGGCCTATTGGGGCCAGGGGACC CAGGTCACCGTCTCCAGCGGCCGCTACCCGTACGACGTTCCGGACTACGGTTCC GGCCGAGCATAG (SEQ ID NO:97) |
| R2DP67 | CAGGTGCAGCTGCAGGAGTCTGGGGGAGGCTTGGTGCAGGCTGGAGGGTCTCTG AGACTCTCCTGTGCAGCCTCTGGAAGTCCCTTCAGTATCGGTACCATGGCCTGGT ACCGCCAGGCTCCAGGGAATCAGCGCGCATCGGTCGCAGCTATTACTAGGGGTG GTGCGACAACCTATGCAAACTCCGTGAAGGGCCGATTCACCGTCTCTAGAGACAA CGCCAAGAACACAGTGTATCTGCAAATGAACAGCCTGAAACCTGAGGACACGGCC GTCTATTATTGTAGTGTCTGGGCCGGCGATAGGGCCTTCTGGGGCCAGGGGACC CAGGTCACCGTCTCCAGCGGCCGCTACCCGTACGACGTTCCGGACTACGGTTCC GGCCGAGCATAG (SEQ ID NO:98) |
| R2DP34 | CAGGTGCAGCTGCAGGAGTCTGGAGGAGGCTTGGTGCAGGCTGGGGGGTCTCTG AGACTCTCCTGTGCAGCCTCTGGAAGCGCCACGCGTATCAATGTCATGAGCTGGT ACCGCCAGGCTCCCGGGAAAAAGCGCGAGTCGGTCGCAGTTGTAACGAGTGGTG GTAGCACAAACTATTTAGCGTCCGTGAAGGGCCGATTCACCATCTCCAGAGACAA CGCCAAGAACACAGTGTATCTGCAAATGAACAGCCTGAAACCTGAGGACACGGCC GTCTATTACTGTGCCGCGGTCGAGGATGAGTATGTCTACTGGGGCAAGGGGACCC AGGTCACCGTCTCCAGCGGCCGCTACCCGTACGACGTTCCGGACTACGGTTCCG GCCGAGCATAG (SEQ ID NO:99) |
| R2DP78 | CAGGTGCAGCTGCAGGAGTCTGGGGGAGGCTTGGTACAGGCTGGGGGGTCTCTG AGACTCTCCTGTGCAGCCTCTGGAATCACCTTCACGCTCAATGTCATGGGCTGGTA CCGCCAGGCTCCAGGGAAGCAGCGCGAGGCGGTCGCAACTATTAGTAATGGTGC GATTAGCAACTATGCAAACTCCGTGAAGGGCCGATTCACCATCTCCAGAGACAAC GCCAAGAACACAGTGTATCTGCAAATGAACAGCCTGAAACCTGAAGACACGGCCG TCTATTATTGTGCTACAGTGATCGGAGGTTCCAGATTTGCAGGCCAGGGGACCCA GGTCACCGTCTCCAGCGGCCGCTACCCGTACGACGTTCCGGACTACGGTTCCGG CCGAGCATAG (SEQ ID NO:100) |
| R2DP12 | CAGGTGCAGCTGCAGGAGTCTGGAGGAGGCTTGGTGCAGGCTGGGGGGTCTCTG AGACTCTCCTGCGCAGCCTCTGGAAGCACCAGCAGCTTCAATGTCATGGCCTGGT ACCACCAGGCTCCAGGGAAGCAGCGCGAGTCGGTCGCAAGTATTACTAGTGGTG GTATCACAGTTTATAGAGACTCCGTGAAGGGCCGCTTCGCCATCTCAAGTGACAAC GCCAAGAACACGGTGTTTCTGCAAATGAACAGCCTGAAACCTGAGGACACAGGCG TCTATTACTGTGGGGGGGTAGTAGATGGGCGAGTCTACCGGGGCCAGGGGACCC AGGTCACCGTCTCCAGCGGCCGCTACCCGTACGACGTTCCGGACTACGGTTCCG GCCGAGCATAG (SEQ ID NO:101) |
| R2DP57 | CAGGTGCAGCTGCAGGAGTCTGGAGGAGGATTGGTGCAGGCTGGGGGGTCTCTG AGACTCTCCTGCGCAGTTTCTGGAACCACCAGCAGTTTCAATGTCATGAACTGGCA CCACCAGGCTCCAGGGAAGCAGCGCGAGTCGGTCGCAAGTATTACTAGTGGTGG TAGCACACTTTATAGAGACTCCGTGAAGGGCCGCCTCACCATCTCCAGAGACAAC GCCAAGAACGCGGTGTATCTGCAAATGAACAGCCTGAAACCTGAGGACACAGGCG TCTATTACTGTGGGGGAGTAGTCGGTGGGCGAGTCTACTGGGGCCAGGGGACCC AGGTCACCGTCTCCAGCGGCCGCTACCCGTACGACGTTCCGGACTACGGTTCCG GCCGAGCATAG (SEQ ID NO:102) |
| R2DP62 | CAGGTGCAGCTGCAGGAGTCTGGGGGAGGCTTGGTGCAGGCTGGGGGGTCTCT GAGACTCTCCTGTGCAGCCTCGGGAAGCATCGTCAGTAGCAATGCCATGAGCTGG TACCGCCAGGCTCCAGGGAAGCAGCGCGAGTCGGTCGCAGCTATTGCGAATAGT GGCATAACAACGTATCGAGACTCCGTGAAGGGCCGATTCACCATCTCCAGAGACA ACACCAAGAACACAATGTATCTGCAAATGAACAGTCTGAGACCTGAAGACACGGC CGTCTATTACTGTGCAGCCATTTTTAATCAACAATTTGCTTGGGGCCAGGGGACCC AGGTCACCGTCTCCAGCGGCCGCTACCCGTACGACGTTCCGGACTACGGTTCCG GCCGAGCATAG (SEQ ID NO:103) |
| R2DP35 | CAGGTGCAGCTGCAGGAGTCTGGGGGAGGCTTGGTGCAGCCTGGGGGGTCTCTG ACACTCTCCTGTGCCGCCTCTGGAGTCATGTTCAGTACCGGTGTCATGGGCTGGT ACCGTCAGGCTCCAGGGAAGCAGCGCGAGGCGGTTGCAGCCATTACTAGCGGCG GTCTCACAACCTATAAAGACTCCGTGAAGGGCCGATTCGCCATCTCCAGAGACAA CACCAAGAGTACGGGGTATCTGCAAATGAACCGCCTGAGACCTGAGGACACGGC CGTCTATTACTGCAATGTCCGGGGCTACTGGGGTCAGGGGACCCAGGTCACCGTC TCCAGCGGCCGCTACCCGTACGACGTTCCGGACTACGGTTCCGGCCGAGCATAG (SEQ ID NO:104) |
| R2DP55 | CAGGTGCAGCTGCAGGAGTCTGGGGGAGGCTTGGTGCAGGCTGGGGGGTCTCT GAGAGTCTCCTGTGCAACCTCTGGATTCACTTTCGATGACCATGCAATAGGCTGGT TCCGCCAGGCCCCAGGGAAGGAGCGCGAGGCGGTCTCATGTCTTAGTAATAGTG ATGGTACTACATACTATACAGACTCCGTGAAGGGCCGATTCACCGCCTCCAGTGA CAACGCCAAGAAAACGGGGTATCTACAAATGAAGAGCCTGAAACCTGAGGACACG GCCGTTTATTACTGTGCAATTTGCCATTTTAGGGAAAGGGACGGCAGTGCCTGGTT TGAGGGTTCCTGGGGCCAGGGGACCCAGGTCACCGTCTCCAGCGGCCGCTACCC GTACGACGTTCCGGACTACGGTTCCGGCCGAGCATAG |
| | (SEQ ID NO:105) |

CDRs of isolated single domain antibodies according to the invention have beendetermined according to IMGT (International ImMunoGeneTics information system aminoacid numbering (http://imgt.cines.fr/)).

The present invention relates to a kit for inhibiting the deNEDDylating enzyme NEDP1, in particular for the prevention and/or treatment of neurodegenerative diseases such as Amyotrophic Lateral Sclerosis and/or Frontotemporal Degeneration, said kit comprising at least one isolated single domain antibody specific for the deNEDDylating enzyme NEDP1chosen from:
- an isolated single domain antibody called AbR2DP44 which comprises CDR-H1 of sequence SEQ ID NO:1, CDR-H2 of sequence SEQ ID NO:2 and CDR-H3 of sequence SEQ ID NO:3;
- an isolated single domain antibody called AbR2DP58 which comprises CDR-H1 of sequence SEQ ID NO:4, CDR-H2 of sequence SEQ ID NO:5 and CDR-H3 of sequence SEQ ID NO:6;
- an isolated single domain antibody called AbR2DP61 which comprises CDR-H1 of sequence SEQ ID NO:7, CDR-H2 of sequence SEQ ID NO:8 and CDR-H3 of sequence SEQ ID NO:9;
- an isolated single domain antibody called AbR2DP80 which comprises CDR-H1 of sequence SEQ ID NO:10, CDR-H2 of sequence SEQ ID NO:11 and CDR-H3 of sequence SEQ ID NO:12;
- an isolated single domain antibody called AbR2DP16 which comprises CDR-H1 of sequence SEQ ID NO:13, CDR-H2 of sequence SEQ ID NO:14 and CDR-H3 of sequence SEQ ID NO:15;
- an isolated single domain antibody called AbR2DP23 which comprises CDR-H1 of sequence SEQ ID NO:16, CDR-H2 of sequence SEQ ID NO:17 and CDR-H3 of sequence SEQ ID NO:18;
- an isolated single domain antibody called AbR2DP47 which comprises CDR-H1 of sequence SEQ ID NO:19, CDR-H2 of sequence SEQ ID NO:20 and CDR-H3 of sequence SEQ ID NO:21;
- an isolated single domain antibody called AbR2DP49 which comprises CDR-H1 of sequence SEQ ID NO:22, CDR-H2 of sequence SEQ ID NO:23 and CDR-H3 of sequence SEQ ID NO:24;
- an isolated single domain antibody called AbR2DP69 which comprises CDR-H1 of sequence SEQ ID NO:25, CDR-H2 of sequence SEQ ID NO:26 and CDR-H3 of sequence SEQ ID NO:27;
- an isolated single domain antibody called AbR2DP19 which comprises CDR-H1 of sequence SEQ ID NO:28, CDR-H2 of sequence SEQ ID NO:29 and CDR-H3 of sequence SEQ ID NO:30;
- an isolated single domain antibody called AbR2DP93 which comprises CDR-H1 of sequence SEQ ID NO:31, CDR-H2 of sequence SEQ ID NO:32 and CDR-H3 of sequence SEQ ID NO:33;
- an isolated single domain antibody called AbR2DP11 which comprises CDR-H1 of sequence SEQ ID NO:34, CDR-H2 of sequence SEQ ID NO:35 and CDR-H3 of sequence SEQ ID NO:36;
- an isolated single domain antibody called AbR2DP46 which comprises CDR-H1 of sequence SEQ ID NO:37, CDR-H2 of sequence SEQ ID NO:38 and CDR-H3 of sequence SEQ ID NO:39;
- an isolated single domain antibody called AbR2DP67 which comprises CDR-H1 of sequence SEQ ID NO:40, CDR-H2 of sequence SEQ ID NO:41 and CDR-H3 of sequence SEQ ID NO:42;
- an isolated single domain antibody called AbR2DP34 which comprises CDR-H1 of sequence SEQ ID NO:43, CDR-H2 of sequence SEQ ID NO:44 and CDR-H3 of sequence SEQ ID NO:45;
- an isolated single domain antibody called AbR2DP78 which comprises CDR-H1 of sequence SEQ ID NO:46, CDR-H2 of sequence SEQ ID NO:47 and CDR-H3 of sequence SEQ ID NO:48;
- an isolated single domain antibody called AbR2DP12 which comprises CDR-H1 of sequence SEQ ID NO:49, CDR-H2 of sequence SEQ ID NO:50 and CDR-H3 of sequence SEQ ID NO:51;
- an isolated single domain antibody called AbR2DP57 which comprises CDR-H1 of sequence SEQ ID NO:52, CDR-H2 of sequence SEQ ID NO:53 and CDR-H3 of sequence SEQ ID NO:54;
- an isolated single domain antibody called AbR2DP62 which comprises CDR-H1 of sequence SEQ ID NO:55, CDR-H2 of sequence SEQ ID NO:56 and CDR-H3 of sequence SEQ ID NO:57; and
- an isolated single domain antibody called AbR2DP55 which comprises CDR-H1 of sequence SEQ ID NO:61, CDR-H2 of sequence SEQ ID NO:62 and CDR-H3 of sequence SEQ ID NO:63.

In particular, the kit according to the invention is characterized in that said at least one isolated single domain antibody specific for the deNEDDylating enzyme NEDP1 is chosen from:
- an isolated single domain antibody called AbR2DP44 having the sequence set forth as SEQ ID NO:64 or a sequence at least 85% identical to SEQ ID NO:64;
- an isolated single domain antibody called AbR2DP58 having the sequence set forth as SEQ ID NO:65 or a sequence at least 85% identical to SEQ ID NO:65;
- an isolated single domain antibody called AbR2DP61 having the sequence set forth as SEQ ID NO:66 or a sequence at least 85% identical to SEQ ID NO:66;
- an isolated single domain antibody called AbR2DP80 having the sequence set forth as SEQ ID NO:67 or a sequence at least 85% identical to SEQ ID NO:67;
- an isolated single domain antibody called AbR2DP16 having the sequence set forth as SEQ ID NO:68 or a sequence at least 85% identical to SEQ ID NO:68;
- an isolated single domain antibody called AbR2DP23 having the sequence set forth as SEQ ID NO:69 or a sequence at least 85% identical to SEQ ID NO:69;
- an isolated single domain antibody called AbR2DP47 having the sequence set forth as SEQ ID NO:70 or a sequence at least 85% identical to SEQ ID NO:70;
- an isolated single domain antibody called AbR2DP49 having the sequence set forth as SEQ ID NO:71 or a sequence at least 85% identical to SEQ ID NO:71;
- an isolated single domain antibody called AbR2DP69 having the sequence set forth as SEQ ID NO:72 or a sequence at least 85% identical to SEQ ID NO:72;
- an isolated single domain antibody called AbR2DP19 having the sequence set forth as SEQ ID NO:73 or a sequence at least 85% identical to SEQ ID NO:73;
- an isolated single domain antibody called AbR2DP93 having the sequence set forth as SEQ ID NO:74 or a sequence at least 85% identical to SEQ ID NO:74;
- an isolated single domain antibody called AbR2DP11 having the sequence set forth as SEQ ID NO:75 or a sequence at least 85% identical to SEQ ID NO:75;
- an isolated single domain antibody called AbR2DP46 having the sequence set forth as SEQ ID NO:76 or a sequence at least 85% identical to SEQ ID NO:76;
- an isolated single domain antibody called AbR2DP67 having the sequence set forth as SEQ ID NO:77 or a sequence at least 85% identical to SEQ ID NO:77;
- an isolated single domain antibody called AbR2DP34 having the sequence set forth as SEQ ID NO:78 or a sequence at least 85% identical to SEQ ID NO:78;
- an isolated single domain antibody called AbR2DP78 having the sequence set forth as SEQ ID NO:79 or a sequence at least 85% identical to SEQ ID NO:79;
- an isolated single domain antibody having the sequence set forth as SEQ ID NO:80 or a sequence at least 85% identical to SEQ ID NO:80;
- an isolated single domain antibody called AbR2DP57 having the sequence set forth as SEQ ID NO:81 or a sequence at least 85% identical to SEQ ID NO:81;
- an isolated single domain antibody called AbR2DP62 having the sequence set forth as SEQ ID NO:82 or a sequence at least 85% identical to SEQ ID NO:82;
   and
- an isolated single domain antibody called AbR2DP55 having the sequence set forth as SEQ ID NO:84 or a sequence at least 85% identical to SEQ ID NO:84.

The present invention further relates to at least one isolated single domain antibody chosen from:
- an isolated single domain antibody called AbR2DP44 which comprises CDR-H1 of sequence SEQ ID NO:1, CDR-H2 of sequence SEQ ID NO:2 and CDR-H3 of sequence SEQ ID NO:3;
- an isolated single domain antibody called AbR2DP58 which comprises CDR-H1 of sequence SEQ ID NO:4, CDR-H2 of sequence SEQ ID NO:5 and CDR-H3 of sequence SEQ ID NO:6;
- an isolated single domain antibody called AbR2DP61 which comprises CDR-H1 of sequence SEQ ID NO:7, CDR-H2 of sequence SEQ ID NO:8 and CDR-H3 of sequence SEQ ID NO:9;
- an isolated single domain antibody called AbR2DP80 which comprises CDR-H1 of sequence SEQ ID NO:10, CDR-H2 of sequence SEQ ID NO:11 and CDR-H3 of sequence SEQ ID NO:12;
- an isolated single domain antibody called AbR2DP16 which comprises CDR-H1 of sequence SEQ ID NO:13, CDR-H2 of sequence SEQ ID NO:14 and CDR-H3 of sequence SEQ ID NO:15;
- an isolated single domain antibody called AbR2DP23 which comprises CDR-H1 of sequence SEQ ID NO:16, CDR-H2 of sequence SEQ ID NO:17 and CDR-H3 of sequence SEQ ID NO:18;
- an isolated single domain antibody called AbR2DP47 which comprises CDR-H1 of sequence SEQ ID NO:19, CDR-H2 of sequence SEQ ID NO:20 and CDR-H3 of sequence SEQ ID NO:21;
- an isolated single domain antibody called AbR2DP49 which comprises CDR-H1 of sequence SEQ ID NO:22, CDR-H2 of sequence SEQ ID NO:23 and CDR-H3 of sequence SEQ ID NO:24;
- an isolated single domain antibody called AbR2DP69 which comprises CDR-H1 of sequence SEQ ID NO:25, CDR-H2 of sequence SEQ ID NO:26 and CDR-H3 of sequence SEQ ID NO:27;
- an isolated single domain antibody called AbR2DP19 which comprises CDR-H1 of sequence SEQ ID NO:28, CDR-H2 of sequence SEQ ID NO:29 and CDR-H3 of sequence SEQ ID NO:30;
- an isolated single domain antibody called AbR2DP93 which comprises CDR-H1 of sequence SEQ ID NO:31, CDR-H2 of sequence SEQ ID NO:32 and CDR-H3 of sequence SEQ ID NO:33;
- an isolated single domain antibody called AbR2DP11 which comprises CDR-H1 of sequence SEQ ID NO:34, CDR-H2 of sequence SEQ ID NO:35 and CDR-H3 of sequence SEQ ID NO:36;
- an isolated single domain antibody called AbR2DP46 which comprises CDR-H1 of sequence SEQ ID NO:37, CDR-H2 of sequence SEQ ID NO:38 and CDR-H3 of sequence SEQ ID NO:39;
- an isolated single domain antibody called AbR2DP67 which comprises CDR-H1 of sequence SEQ ID NO:40, CDR-H2 of sequence SEQ ID NO:41 and CDR-H3 of sequence SEQ ID NO:42;
- an isolated single domain antibody called AbR2DP34 which comprises CDR-H1 of sequence SEQ ID NO:43, CDR-H2 of sequence SEQ ID NO:44 and CDR-H3 of sequence SEQ ID NO:45;
- an isolated single domain antibody called AbR2DP78 which comprises CDR-H1 of sequence SEQ ID NO:46, CDR-H2 of sequence SEQ ID NO:47 and CDR-H3 of sequence SEQ ID NO:48;
- an isolated single domain antibody called AbR2DP12 which comprises CDR-H1 of sequence SEQ ID NO:49, CDR-H2 of sequence SEQ ID NO:50 and CDR-H3 of sequence SEQ ID NO:51;
- an isolated single domain antibody called AbR2DP57 which comprises CDR-H1 of sequence SEQ ID NO:52, CDR-H2 of sequence SEQ ID NO:53 and CDR-H3 of sequence SEQ ID NO:54;
- an isolated single domain antibody called AbR2DP62 which comprises CDR-H1 of sequence SEQ ID NO:55, CDR-H2 of sequence SEQ ID NO:56 and CDR-H3 of sequence SEQ ID NO:57;
   ; and
- an isolated single domain antibody called AbR2DP55 which comprises CDR-H1 of sequence SEQ ID NO:61, CDR-H2 of sequence SEQ ID NO:62 and CDR-H3 of sequence SEQ ID NO:63.
for use for inhibiting the deNEDDylating enzyme NEDP1.

In one embodiment, the at least one isolated single domain antibody for use according to the invention is for use for the prevention and/or treatment of neurodegenerative disease, more particularly to prevent and/or treat Amyotrophic Lateral Sclerosis and Frontotemporal Degeneration.

In particular, the at least one isolated single domain antibody for use according to the invention is characterized in that said at least one isolated single domain antibody is chosen from:
- an isolated single domain antibody called AbR2DP44 having the sequence set forth as SEQ ID NO:64 or a sequence at least 85% identical to SEQ ID NO:64;
- an isolated single domain antibody called AbR2DP58 having the sequence set forth as SEQ ID NO:65 or a sequence at least 85% identical to SEQ ID NO:65;
- an isolated single domain antibody called AbR2DP61 having the sequence set forth as SEQ ID NO:66 or a sequence at least 85% identical to SEQ ID NO:66;
- an isolated single domain antibody called AbR2DP80 having the sequence set forth as SEQ ID NO:67 or a sequence at least 85% identical to SEQ ID NO:67;
- an isolated single domain antibody called AbR2DP16 having the sequence set forth as SEQ ID NO:68 or a sequence at least 85% identical to SEQ ID NO:68;
- an isolated single domain antibody called AbR2DP23 having the sequence set forth as SEQ ID NO:69 or a sequence at least 85% identical to SEQ ID NO:69;
- an isolated single domain antibody called AbR2DP47 having the sequence set forth as SEQ ID NO:70 or a sequence at least 85% identical to SEQ ID NO:70;
- an isolated single domain antibody called AbR2DP49 having the sequence set forth as SEQ ID NO:71 or a sequence at least 85% identical to SEQ ID NO:71;
- an isolated single domain antibody called AbR2DP69 having the sequence set forth as SEQ ID NO:72 or a sequence at least 85% identical to SEQ ID NO:72;
- an isolated single domain antibody called AbR2DP19 having the sequence set forth as SEQ ID NO:73 or a sequence at least 85% identical to SEQ ID NO:73;
- an isolated single domain antibody called AvR2DP93 having the sequence set forth as SEQ ID NO:74 or a sequence at least 85% identical to SEQ ID NO:74;
- an isolated single domain antibody called AbR2DP11 having the sequence set forth as SEQ ID NO:75 or a sequence at least 85% identical to SEQ ID NO:75;
- an isolated single domain antibody called AbR2DP46 having the sequence set forth as SEQ ID NO:76 or a sequence at least 85% identical to SEQ ID NO:76;
- an isolated single domain antibody called AbR2DP67 having the sequence set forth as SEQ ID NO:77 or a sequence at least 85% identical to SEQ ID NO:77;
- an isolated single domain antibody called AbR2DP34 having the sequence set forth as SEQ ID NO:78 or a sequence at least 85% identical to SEQ ID NO:78;
- an isolated single domain antibody called AbR2DP78 having the sequence set forth as SEQ ID NO:79 or a sequence at least 85% identical to SEQ ID NO:79;
- an isolated single domain antibody called AbR2DP12 having the sequence set forth as SEQ ID NO:80 or a sequence at least 85% identical to SEQ ID NO:80;
- an isolated single domain antibody called AbR2DP57 having the sequence set forth as SEQ ID NO:81 or a sequence at least 85% identical to SEQ ID NO:81;
- an isolated single domain antibody called AbR2DP62 having the sequence set forth as SEQ ID NO:82 or a sequence at least 85% identical to SEQ ID NO:82; and
- an isolated single domain antibody called AbR2DP55 having the sequence set forth as SEQ ID NO:84 or a sequence at least 85% identical to SEQ ID NO:84

In one embodiment, the kit according to the invention or the at least one isolated single domain antibody for use according to the invention, is characterized in that said at least one isolated single domain antibody specific for the deNEDDylating enzyme NEDP1 is chosen from AbR2DP80, AbR2DP67, AbR2DP34, AbR2DP78, AbR2DP57, AbR2DP12, AbR2PD46 and AbR2DP47.

In particular, in one embodiment, said at least one isolated single domain antibody specific for the deNEDDylating enzyme NEDP1 is chosen from AbR2DP12, AbR2PD46 and AbR2DP47.

In particular, in another embodiment, said at least one isolated single domain antibody specific for the deNEDDylating enzyme NEDP1 is chosen from AbR2DP80, AbR2DP67, AbR2DP34, AbR2DP78, AbR2DP57.

In the context of the present invention, the mere fact that certain features are recited in different embodiments does not indicate that a combination of these features cannot be used.

As used herein "at least one isolated single domain antibody" means 1 or more isolated single domain antibody, in particular 1, 2, 3, 4, 5, 6, 7, 8, 9, 10, 11, 12, 13, 14, 15, 16, 17, 18, 19, 20 or 21 isolated single domain antibodies.

As used herein the term "single domain antibody" has its general meaning in the art and refers to the single heavy chain variable domain of antibodies that are naturally devoid of light chains of the type that can be found in Camelid mammals. Such single domain antibody is also called VHH or "nanobody^{®}". For a general description of (single) domain antibodies, reference can be made to EP 0 368 684, Ward et al. (Nature 1989 Oct 12; 341 (6242): 544-6), Holt et al., Trends Biotechnol., 2003, 21(11):484-490; and WO 06/030220, WO 06/003388. The nanobody has a molecular weight approximately one-tenth that of a human IgG molecule, and the protein has a physical diameter of only a few nanometers. One consequence of the small size is the ability of camelid nanobodies to bind to antigenic sites that are functionally invisible to larger antibody proteins, i.e. , camelid nanobodies are useful as reagents to detect antigens that are otherwise cryptic using classical immunological techniques, and as possible therapeutic agents. Thus, yet another consequence of small size is that a nanobody can inhibit as a result of binding to a specific site in a groove or narrow cleft of a target protein, and hence can serve in a capacity that more closely resembles the function of a classical low molecular weight drug than that of a classical antibody. The low molecular weight and compact size further result in nanobodies being extremely thermostable, stable to extreme pH and to proteolytic digestion, and poorly antigenic. Another consequence is that nanobodies readily move from the circulatory system into tissues, and even cross the blood-brain barrier and can treat disorders that affect nervous tissue. Nanobodies can further facilitate drug transport across the blood brain barrier. See U.S. patent application 20040161738 published August 19, 2004. These features combined with the low antigenicity to humans indicate great therapeutic potential.The amino acid sequence and structure of a single domain antibody can be considered to be comprised of four framework regions or "FRs" which are referred to in the art and herein as "Framework region 1" or "FR1 "; as "Framework region 2" or "FR2"; as "Framework region 3 " or "FR3"; and as "Framework region 4" or "FR4" respectively; which framework regions are interrupted by three complementary determining regions or "CDRs", which are referred to in the art as "Complementarity Determining Region 1" for "CDR1"; as "Complementarity Determining Region 2" or "CDR2" and as "Complementarity Determining Region 3" or "CDR3", respectively. Accordingly, the single domain antibody can be defined as an amino acid sequence with the general structure : FR1 - CDR1 - FR2 - CDR2 - FR3 - CDR3 - FR4 in which FR1 to FR4 refer to framework regions 1 to 4 respectively, and in which CDR1 to CDR3 refer to the complementarity determining regions 1 to 3. In the context of the invention, the amino acid residues of the single domain antibody are numbered according to the general numbering for VH domains given by the International ImMunoGeneTics information system aminoacid numbering (http://imgt.cines.fr/).

The above-mentioned sequences are amino acids sequences.

A sequence "at least 75% identical" to a reference sequence is a sequence having, on its entire length, 75%, or more, in particular 80%, 85%, 90%, 91%, 92%, 93%, 94%, 95%, 96%, 97%, 98% or 99% sequence identity with the entire length of the reference sequence.

A percentage of "sequence identity" may be determined by comparing the two sequences, optimally aligned over a comparison window, wherein the portion of the polynucleotide or polypeptide sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison and multiplying the result by 100 to yield the percentage of sequence identity. Optimal alignment of sequences for comparison is conducted byglobal pairwise alignment, e.g. using the algorithm of Needleman and Wunsch J. Mol. Biol. 48:443 (1970). The percentage of sequence identity can be readily determined for instance using the program Needle, with the BLOSUM62 matrix, and the following parameters gap-open=10, gap-extend=0.5.

According to the invention a sequence having at least 85% of identity with a second amino acid sequence means that the first sequence has 85; 86; 87; 88; 89; 90; 91; 92; 93; 94; 95; 96; 97; 98; or 99% of identity with the second amino acid sequence. Amino acid sequence identity is typically determined using a suitable sequence alignment algorithm and default parameters, such as BLAST P (Karlin and Altschul, 1990).

Camel Ig can be modified by genetic engineering to yield a small protein having high affinity for a target, resulting in a low molecular weight antibody-derived protein known as a "nanobody" or "VHH". See U.S. patent number 5,759,808 issued June 2, 1998; see also Stijlemans, B. et al. , 2004 J Biol Chem 279: 1256-1261 ; Dumoulin, M. et a/. , 2003 Nature 424: 783-788; Pleschberger, M. et al. 2003 Bioconjugate Chem 14: 440- 448; Cortez-Retamozo, V. et al. 2002 Int J Cancer 89: 456-62; and Lauwereys, M. et al. 1998 EMBO J 17: 3512-3520. Engineered libraries of camelid antibodies and antibody fragments are commercially available, for example, from Ablynx, Ghent, Belgium. In certain embodiments herein, the llamas antibody or nanobody is naturally produced in the camelid animal, i.e., is produced by the camelid following immunization with NEDP1, using techniques described herein for other antibodies. Alternatively, the NEDP1-binding camelid nanobody is engineered, i.e., produced by selection for example from a library of phage displaying appropriately mutagenized camelid nanobody proteins using panning procedures with NEDP1 enzymes a target.

In some embodiments, the single domain antibody is a "humanized" single domain antibody.

As used herein the term "humanized" refers to a single domain antibody of the invention wherein an amino acid sequence that corresponds to the amino acid sequence of a naturally occurring VHH domain has been "humanized", i.e. by replacing one or more amino acid residues in the amino acid sequence of said naturally occurring VHH sequence (and in particular in the framework sequences) by one or more of the amino acid residues that occur at the corresponding position(s) in a VH domain from a conventional chain antibody from a human being. Methods for humanizing single domain antibodies are well known in the art. Typically, the humanizing substitutions should be chosen such that the resulting humanized single domain antibodies still retain the favourable properties of single domain antibodies of the invention. The one skilled in the art is able to determine and select suitable humanizing substitutions or suitable combinations of humanizing substitutions.

According to the invention, the single domain antibodies of the invention may be produced by conventional automated peptide synthesis methods, by mRNA synthesis and injection or by recombinant expression. General principles for designing and making proteins are well known to those of skill in the art. The single domain antibodies of the invention may be synthesized in solution or on a solid support in accordance with conventional techniques. Various automatic synthesizers are commercially available and can be used in accordance with known protocols as described in Stewart and Young; Tam et al., 1983; Merrifield, 1986 and Barany and Merrifield, Gross and Meienhofer, 1979. The single domain antibodies of the invention may also be synthesized by solid-phase technology employing an exemplary peptide synthesizer such as a Model 433A from Applied Biosystems Inc. The purity of any given protein; generated through automated peptide synthesis or through recombinant methods may be determined using reverse phase HPLC analysis. Chemical authenticity of each peptide may be established by any method well known to those of skill in the art. As an alternative to automated peptide synthesis, recombinant DNA technology may be employed wherein a nucleotide sequence which encodes a protein of choice is inserted into an expression vector, transformed or transfected into an appropriate host cell and cultivated under conditions suitable for expression as described herein below. Recombinant methods are especially preferred for producing longer polypeptides.

According to the invention, the single domain antibodies of the invention are encoded by a nucleic acid sequence. Said nucleic acid sequences are mentioned in Table 2 above.

Typically, said nucleic acid sequence may be included in any suitable vector, such as a plasmid, cosmid, episome, artificial chromosome, phage or a viral vector.

As used herein, the terms "vector", "cloning vector" and "expression vector" mean the vehicle by which a DNA or RNA sequence (e.g. a foreign gene) can be introduced into a host cell, so as to transform the host and promote expression (e.g. transcription and translation) of the introduced sequence. Such vectors may comprise regulatory elements, such as a promoter, enhancer, terminator and the like, to cause or direct expression of said antibody upon administration to a subject. Examples of promoters and enhancers used in the expression vector for animal cell include early promoter and enhancer of SV40 (Mizukami T. et al. 1987), LTR promoter and enhancer of Moloney mouse leukemia virus (Kuwana Y et al. 1987), promoter (Mason JO et al. 1985) and enhancer (Gillies SD et al. 1983) of immunoglobulin H chain and the like. Any expression vector for animal cell can be used, so long as a gene encoding the human antibody C region can be inserted and expressed. Examples of suitable vectors include pAGE107 (Miyaji H et al. 1990), pAGE103 (Mizukami T et al. 1987), pHSG274 (Brady G et al. 1984), pKCR (O'Hare K et al. 1981), pSG1 beta d2-4-(Miyaji H et al. 1990) and the like. Other examples of plasmids include replicating plasmids comprising an origin of replication, or integrative plasmids, such as for instance pUC, pcDNA, pBR, and the like. Other examples of viral vector include adenoviral, retroviral, herpes virus and AAV vectors. Such recombinant viruses may be produced by techniques known in the art, such as by transfecting packaging cells or by transient transfection with helper plasmids or viruses. Typical examples of virus packaging cells include PA317 cells, PsiCRIP cells, GPenv+ cells, 293 cells, etc. Detailed protocols for producing such replication-defective recombinant viruses may be found for instance in WO 95/14785, WO 96/22378, US 5,882,877, US 6,013,516, US 4,861,719, US 5,278,056 and WO 94/19478.

In the context of the invention, a host cell can be transfected, infected or transformed by a nucleic acid and/or a vector as described above.

The term "transformation" means the introduction of a "foreign" (i.e. extrinsic or extracellular) gene, DNA or RNA sequence to a host cell, so that the host cell will express the introduced gene or sequence to produce a desired substance, typically a protein or enzyme coded by the introduced gene or sequence. A host cell that receives and expresses introduced DNA or RNA bas been "transformed".

The nucleic acids according to the invention may be used to produce a single domain antibody of the present invention in a suitable expression system. The term "expression system" means a host cell and compatible vector under suitable conditions, e.g. for the expression of a protein coded for by foreign DNA carried by the vector and introduced to the host cell. Common expression systems include *E. coli* host cells and plasmid vectors, insect host cells and Baculovirus vectors, and mammalian host cells and vectors. Other examples of host cells include, without limitation, prokaryotic cells (such as bacteria) and eukaryotic cells (such as yeast cells, mammalian cells, insect cells, plant cells, etc.). Specific examples include *E.coli, Kluyveromyces* or *Saccharomyces* yeasts, mammalian cell lines (e.g., Vero cells, CHO cells, 3T3 cells, COS cells, etc.) as well as primary or established mammalian cell cultures (e.g., produced from lymphoblasts, fibroblasts, embryonic cells, epithelial cells, nervous cells, adipocytes, etc.). Examples also include mouse SP2/0-Ag14 cell (ATCC CRL1581), mouse P3X63-Ag8.653 cell (ATCC CRL1580), CHO cell in which a dihydrofolate reductase gene (hereinafter referred to as "DHFR gene") is defective (Urlaub G et al; 1980), rat YB2/3HL.P2.G11.16Ag.20 cell (ATCC CRL1662, hereinafter referred to as "YB2/0 cell"), and the like. The present invention also relates to a method of producing a recombinant host cell expressing at least one single domain antibody according to the invention, said method comprising the steps of: (i) introducing *in vitro* or ex *vivo* a recombinant nucleic acid or a vector as described above into a competent host cell, (ii) culturing *in vitro* or *ex vivo* the recombinant host cell obtained and (iii), optionally, selecting the cells which express and/or secrete said at least one single domain antibody. Such recombinant host cells can be used for the production of single domain antibodies of the present invention.

Furthermore, in the context of the invention, « deNEDDylating enzyme NEDP1 » is used interchangeably with « NEDP1 enzyme » or « NEDP1 » and is a human NEDD8-specific protease.

As mentioned above, the present invention relates to a kit for inhibiting the deNEDDylating enzyme NEDP1, in particular for the prevention and/or treatment of neurodegenerative diseases such as Amyotrophic Lateral Sclerosis and/or Frontotemporal Degeneration, said kit comprising at least one isolated single domain antibody specific for the deNEDDylating enzyme NEDP1 according to the invention.

In the context of the present invention, the term "kit" means two or more components one of which corresponding to the at least one single domain antibody or pharmaceutical composition of the invention - packaged together in a container, recipient or otherwise. A kit can hence be described as a set of products and/or utensils that are sufficient to achieve a certain goal, which can be marketed as a single unit.

The kit may comprise one or more recipients (such as vials, ampoules, containers, syringes, bottles, bags) of any appropriate shape, size and material (preferably waterproof, e.g. plastic or glass) containing the at least one single domain antibody of the invention or the pharmaceutical composition of the invention in an appropriate dosage for administration. The kit may additionally contain directions for use (e.g. in the form of a leaflet or instruction manual), means for administering the at least one single domain antibody of the invention such as a syringe, pump, infuser or the like, means for reconstituting the at least one single domain antibody of the invention and/or means for diluting at least one single domain antibody of the invention.

The invention also provides kits for a single-dose administration unit. The kit of the invention may also contain a first recipient comprising a dried /lyophilized at least one single domain antibody of the invention and a second recipient comprising an aqueous formulation. In certain embodiments of this invention, kits containing single-chambered and multi-chambered pre-filled syringes (e.g., liquid syringes and lyosyringes) are provided.

In one embodiment, such kit is sufficient for one complete treatment. Parts of the kit of the invention can be packaged individually in vials or bottles or in combination in containers or multicontainer units. The manufacture of the kits follows preferably standard procedures which are known to the person skilled in the art.

Furthermore, the invention relates to a kit as described hereinbefore and written instructions for the sequential use thereof in accordance with the methods of the present invention. Said kit may further comprise a label or imprint indicating that the contents can be used for inhibiting the deNEDDylating enzyme NEDP1, and/or for the prevention and/or treatment of neurodegenerative diseases.

As also mentioned above, the at least one single domain antibody of the invention is for use for inhibiting the deNEDDylating enzyme NEDP1.

In one embodiment, the invention further relates to a method for inhibiting the deNEDDylating enzyme NEDP1, comprising the administration of at least one single domain antibody of the invention. In one embodiment, an effective amount of said at least one single domain antibody of the invention is administered, more particularly a therapeutically effective amount.

In particular, the activity of deNEDDylating enzyme NEDP1 is inhibited in *in vitro* reactions, in cell extracts and in live cells.

As a consequence, the at least one single domain antibodies of the invention is administered either *in vitro* to cells or *in vivo.*

As also mentioned above, the at least one single domain antibody of the invention is for use for the prevention and/or treatment of neurodegenerative diseases.

In one embodiment, the invention further relates to a method to prevent and/or treat a neurodegenerative disease, comprising the administration of at least one single domain antibody of the invention in a subject in need thereof.

In one embodiment, a therapeutically effective amount of said at least one single domain antibody of the invention is administered.

As used herein, the term "subject" refers to a warm-blooded animal such as a mammal, animal or human, in particular a human, who is afflicted with, or has the potential to be afflicted with one or more diseases and conditions described herein. In particular, said subject is concerned with the need to inhibit the activity of the deNEDDylating enzyme NEDP1 and/or is afflicted or has the potential to be afflicted by a neurodegenerative disease.

The terms "treat", "treating", "treated" or "treatment", as used in the context of the invention, refer to therapeutic treatment wherein the object is to eliminate or lessen symptoms. Beneficial or desired clinical results include, but are not limited to, elimination of symptoms, alleviation of symptoms, diminishment of extent of condition, stabilized (i.e., not worsening) state of condition, delay or slowing of progression of the condition.

The terms "prevent", "prevention", "preventing" or "prevented", as used in the context of the present invention, refer to the prevention of the onset, recurrence or spread of a disease or disorder or infection, or of one or more symptoms thereof. In certain embodiments, the terms refer to the treatment with or administration of a compound provided herein prior to the onset of symptoms, particularly to patients at risk of disease or disorder or infection provided herein. The terms encompass the inhibition or reduction of a symptom of the particular disease or disorder or infection. Subjects with familial history of a disease or disorder or infection in particular are candidates for preventive regimens in certain embodiments. In addition, subjects who have a history of recurring symptoms are also potential candidates for the prevention. In this regard, the term "prevention" may be interchangeably used with the term "prophylactic treatment".

By "neurodegenerative diseases" is meant a disease such as Alzheimer's disease, Creutzfeld-Jakob's disease, Huntington's disease, Parkinson's disease, Retinitis pigmentosa, Spinal muscular atrophy, Cerebellar degeneration, Amyotrophic Lateral Sclerosis and/or Frontotemporal Degeneration.

In particular, the neurodegerative disease is chosen from Amyotrophic Lateral Sclerosis and Frontotemporal Degeneration.

Throughout the instant application, the term "comprising" is to be interpreted as encompassing all specifically mentioned features as well optional, additional, unspecified ones. As used herein, the use of the term "comprising" also discloses the embodiment wherein no features other than the specifically mentioned features are present (i.e. "consisting of").

The identification of the subjects who are in need of treatment of herein-described diseases and conditions is conducted as above mentioned and is well within the ability and knowledge of one skilled in the art. A clinician skilled in the art can readily identify, by the above mentioned techniques, those subjects who are in need of such treatment.

A therapeutically effective amount can be readily determined by the attending diagnostician, as one skilled in the art, by the use of conventional techniques and by observing results obtained under analogous circumstances. In determining the therapeutically effective amount, a number of factors are considered by the attending diagnostician, including, but not limited to: the species of subject; its size, age, and general health; the specific disease involved; the degree of involvement or the severity of the disease; the response of the individual subject; the particular compound administered; the mode of administration; the bioavailability characteristic of the preparation administered; the dose regimen selected; the use of concomitant medication; and other relevant circumstances .

As used herein, a "therapeutically effective amount" refers to an amount which is effective in reducing, eliminating, treating or controlling the symptoms of the herein-described diseases and conditions. The term "controlling" is intended to refer to all processes wherein there may be a slowing, interrupting, arresting, or stopping of the progression of the diseases and conditions described herein, but does not necessarily indicate a total elimination of all disease and condition symptoms, and is intended to include prophylactic treatment and chronic use.

The amount of the single domain antibody according to the invention, which is required to achieve the desired biological effect (i.e inhibit the deNEDDylating enzyme NEDP1 and/or treat and/or prevent a neurodegenerative disease), will vary depending upon a number of factors, including the dosage of the drug to be administered, the chemical characteristics (e.g. hydrophobicity) of the compounds employed, the potency of the single domain antibodies, the type of disease, the diseased state of the patient, and the route of administration.

The single domain antibodies according to the invention can be formulated into pharmaceutical compositions by admixture with a pharmaceutically acceptable vehicle or diluent, in particular a vehicle or diluent suited to the intended use of the single domain antibody.

Pharmaceutically acceptable carriers or excipients that may be used in these compositions include, but are not limited to, virions (adenovirus of lentiviral vectors), ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene- block polymers, polyethylene glycol and wool fat.

The pharmaceutical compositions of the present invention may be administered orally, parenterally, by inhalation spray, topically, rectally, nasally, buccally, vaginally or via an implanted reservoir. The used herein includes subcutaneous, intravenous, intramuscular, intra-articular, intra-nasal, intra-synovial, intrasternal, intrathecal, intrahepatic, intralesional and intracranial injection or infusion techniques. Sterile injectable forms of the compositions of this invention may be aqueous or an oleaginous suspension. These suspensions may be formulated according to techniques known in the art using suitable dispersing or wetting agents and suspending agents. The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono-or diglycerides. Fatty acids, such as oleic acid and its glyceride derivatives are useful in the preparation of injectables, as are natural pharmaceutically acceptable oils, such as olive oil or castor oil, especially in their polyoxyethylated versions. These oil solutions or suspensions may also contain a long-chain alcohol diluent or dispersant, such as carboxymethyl cellulose or similar dispersing agents that are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation.

The pharmaceutical compositions of this invention may be orally administered in any orally acceptable dosage form including, but not limited to, capsules, tablets, aqueous suspensions or solutions. In the case of tablets for oral use, carriers commonly used include lactose and corn starch. Lubricating agents, such as magnesium stearate, are also typically added. For oral administration in a capsule form, useful diluents include, e.g., lactose. When aqueous suspensions are required for oral use, the active ingredient is combined with emulsifying and suspending agents. If desired, certain sweetening, flavoring or coloring agents may also be added. Alternatively, the compositions of this invention may be administered in the form of suppositories for rectal administration. These can be prepared by mixing the agent with a suitable non-irritating excipient that is solid at room temperature but liquid at rectal temperature and therefore will melt in the rectum to release the drug. Such materials include cocoa butter, beeswax and polyethylene glycols. The compositions of this invention may also be administered topically, especially when the target of treatment includes areas or organs readily accessible by topical application, including diseases of the eye, the skin, or the lower intestinal tract. Suitable topical formulations are readily prepared for each of these areas or organs. For topical applications, the compositions may be formulated in a suitable ointment containing the active component suspended or dissolved in one or more carriers. Carriers for topical administration of the compounds of this invention include, but are not limited to, mineral oil, liquid petrolatum, white petrolatum, propylene glycol, polyoxyethylene, polyoxypropylene compound, emulsifying wax and water. Alternatively, the compositions can be formulated in a suitable lotion or cream containing the active components suspended or dissolved in one or more pharmaceutically acceptable carriers. Suitable carriers include, but are not limited to, mineral oil, sorbitan monostearate, polysorbate 60, cetyl esters wax, cetearyl alcohol, 2-octyldodecanol, benzyl alcohol and water. Topical application for the lower intestinal tract can be performed in a rectal suppository formulation (see above) or in a suitable enema formulation. Patches may also be used. The compositions of this invention may also be administered by nasal aerosol or inhalation. Such compositions are prepared according to techniques well known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents.

In certain embodiments, the use of liposomes and/or nanoparticles is contemplated for the introduction of single domain antibodies into host cells. The formation and use of liposomes and/or nanoparticles are known to those of skill in the art.

Nanocapsules can generally entrap compounds in a stable and reproducible way. To avoid side effects due to intracellular polymeric overloading, such ultrafine particles (sized around 0.1 µm) are generally designed using polymers able to be degraded *in vivo.* Biodegradable polyalkyl-cyanoacrylate nanoparticles that meet these requirements are contemplated for use in the present invention, and such particles may be easily made.

Liposomes are formed from phospholipids that are dispersed in an aqueous medium and spontaneously form multilamellar concentric bilayer vesicles (also termed multilamellar vesicles (MLVs)). MLVs generally have diameters of from 25 nm to 4 µm. Sonication of MLVs results in the formation of small unilamellar vesicles (SUVs) with diameters in the range of 200 to 500 Å, containing an aqueous solution in the core. The physical characteristics of liposomes depend on pH, ionic strength and the presence of divalent cations.

In the scope of the present invention, it has to be understood that "a single domain antibody for use" is equivalent to "the use of a single domain antibody" and in particular that "a single domain antibody, for use in the prevention and/or treatment of" is equivalent to "the use of a single domain antibody, for the prevention and/or treatment of" and to "the use of a single domain antibody for the manufacture of a medicament intended for the prevention and/or treatment of".

The invention will be further illustrated in view of the following figures and examples.

### FIGURES

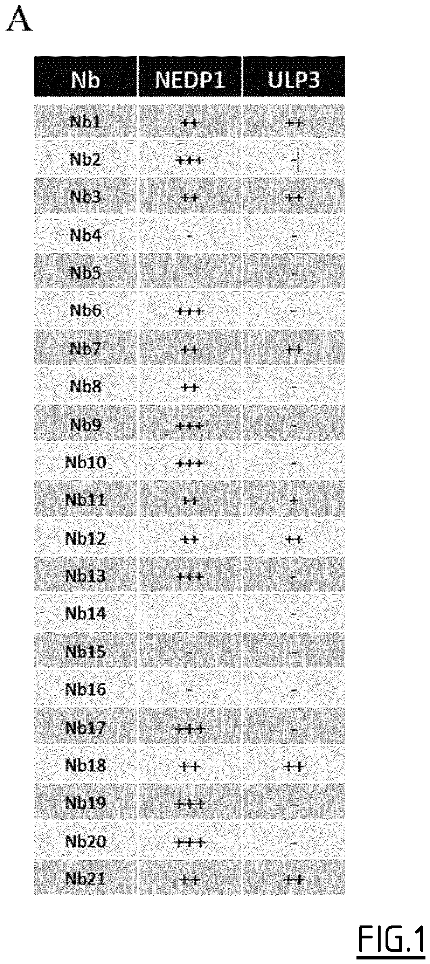

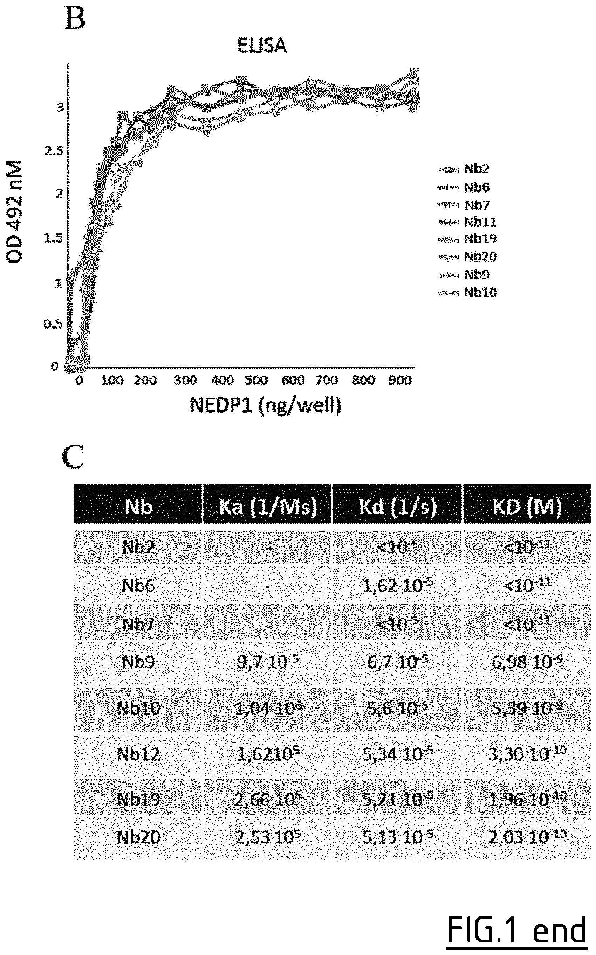

**Figure 1****:** A. Table showing the different Nanobodies isolated and their binding specificity towards the immunogen human NEDP1 and ULP-3, the C. *elegans* homologue of NEDP1 (- poor/no binding, + indicates binding affinity/specificity). B. ELISA test, showing the binding of indicated Nanobodies (300ng) against increasing amounts of coated recombinant human NEDP1. C. Table showing the SPR (Surface Plasmon Resonance) analysis and binding affinities for indicated Nanobodies against human NEDP1.

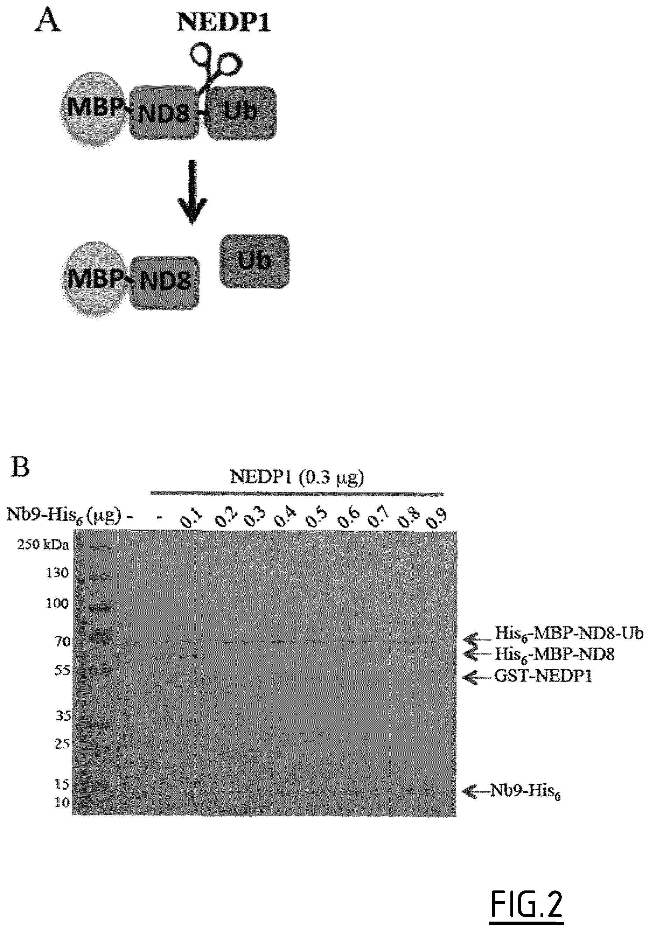

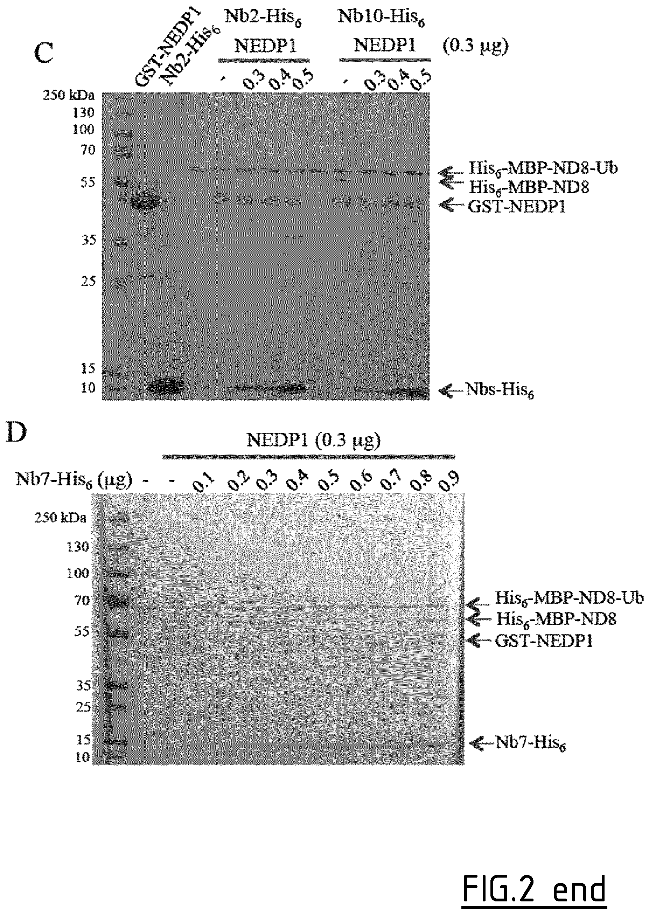

**Figure 2****:** A. Schematic representation of the assay used to determine the effect of Nanobodies on the NEDP1 processing activity for NEDD8. The HiS₆-MBP-NEDD8-ubiquitin fusion protein is incubated with recombinant NEDP1. The production of HiS₆-MBP-NEDD8 is indicative of the processing activity of NEDP1. B. The processing assay was performed where NEDP1 was incubated with HiS₆-MBP-NEDD8-ubiquitin alone or in the presence of increasing amounts of purified Nanobody 9 (AbR2DP46). Reactions were analysed by SDS-PAGE and gels were Coomassie blue stained. Arrows indicate the presence of the processing product HiS₆-MBP-NEDD8 and of the additional reaction components. C. Assay performed as in B but Nanobodies 2 (AbR2DP12) and 10 (AbR2DP47) were used instead. In the first two lanes 1µg of NEDP1 or Nanobody 2-HiS₆ (AbR2DP12) respectively were loaded as control. D. Similar as in B but Nanobody 7 (AbR2DP35) was used instead.

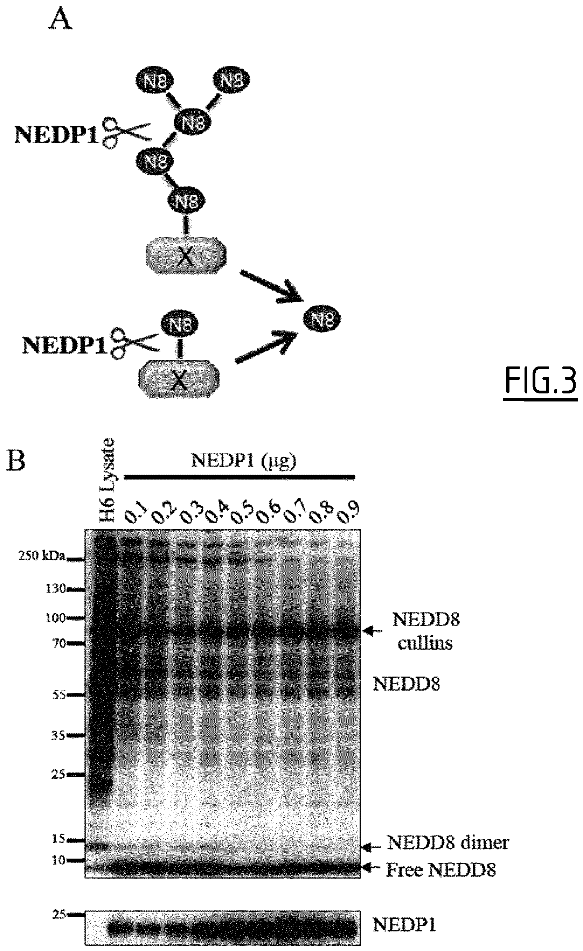

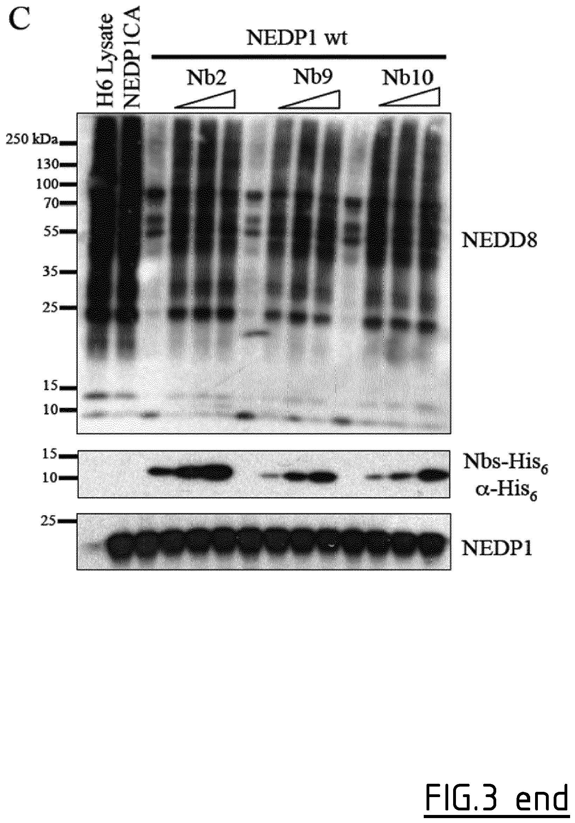

**Figure 3****:** A. Schematic representation of the assay used to determine the effect of Nbs on the deconjugation activity of NEDP1. Extracts from NEDP1 knockout U2OS cells (H6) with high levels of mono- and poly-NEDDylated conjugates were incubated with recombinant NEDP1 in the absence or presence of Nanobodies. The decrease in global NEDDylation and the accumulation of free-NEDD8 are indicative of the NEDD8 deconjugation from substrates. B. Different amounts of recombinant NEDP1 were added in H6 extracts (20µg total protein). Reactions were analysed by western blotting with the indicated antibodies. C. Similar experiment as in B, where 300ng of NEDP1 were added to H6 extracts either alone or with increasing amounts of purified Nanobody 2 (AbR2DP12), 9 (AbR2DP46), 10 (AbR2DP47) (0.1, 0.2, 0.3µg). The catalytic inactive C163A NEDP1 mutant was also used as control for the deconjugating activity of NEDP1.

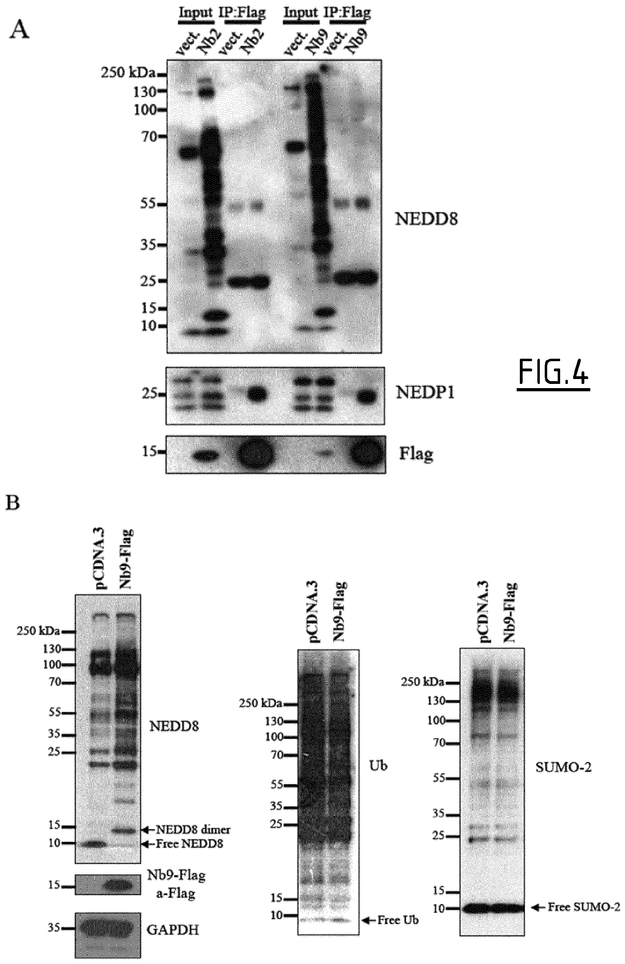

**Figure 4****:** A. Extracts from empty pcDNA3 vector (vect.) or stably expressing Nanobody 2/Nanobody 9-Flag (AbR2DP12/AbR2DP46) U2OS cells were used for anti-Flag immunoprecipitations. Total cell extracts (input) and eluates were used for western blot analysis with the indicated antibodies. B. U2OS cells described above were lysed in 2xSDS buffer and extracts were analysed by western blotting with the indicated antibodies.

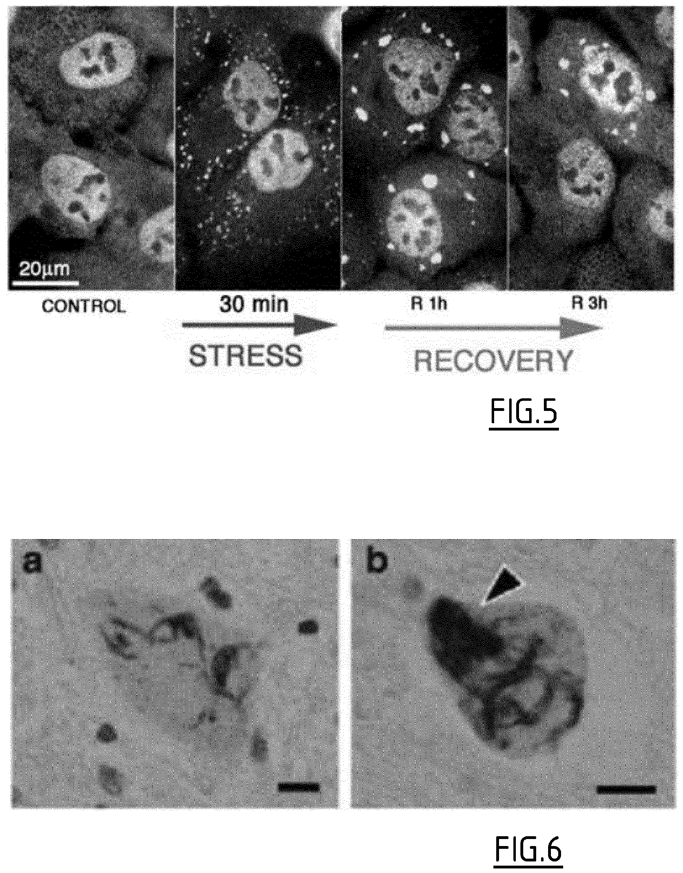

**Figure 5****:** Assembly and disassembly (recovery) of stress-induced (arsenic) stress granules (SGs). Labelling of SGs was performed using antibodies against the SG protein TIA-1. Arrows indicate SGs.
**Figure 6****:** Cytoplasmic aggregates accumulate in ALS. Patient samples stained for SGs (TDP-43 protein). Arrows indicate aberrant inclusions (Mori et al., Acta Neuropathol., 2008).

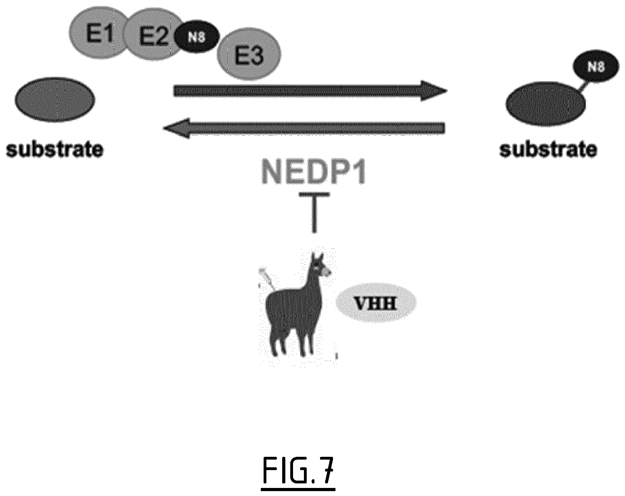

**Figure 7****:** The cycle for NEDD8 (N8) conjugation. NEDP1 removes NEDD8 from substrates. Development of Nanobodies as first-in-class inhibitors for NEDP1.

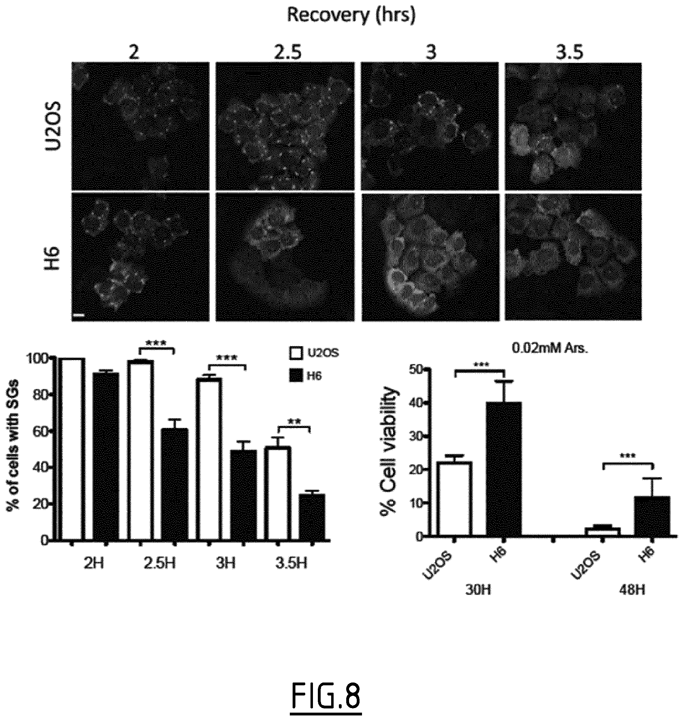

**Figure 8****:** NEDP1 deletion accelerates the elimination of SGs. Control and NEDP1 knockout cells (H6) were exposed to proteotoxic stress (arsenic) before allowed to recover and the elimination of SGs was monitored by live imaging of the SG protein G3BP1 tagged with GFP. Bottom panel (left): Quantitation of the experiment shown in the top panel. Bottom panel (right): Knockout of NEDP1 (H6) improves survival of cells exposed to proteotoxic stress (arsenic). Survival was measured 30 or 48 hrs post treatment.

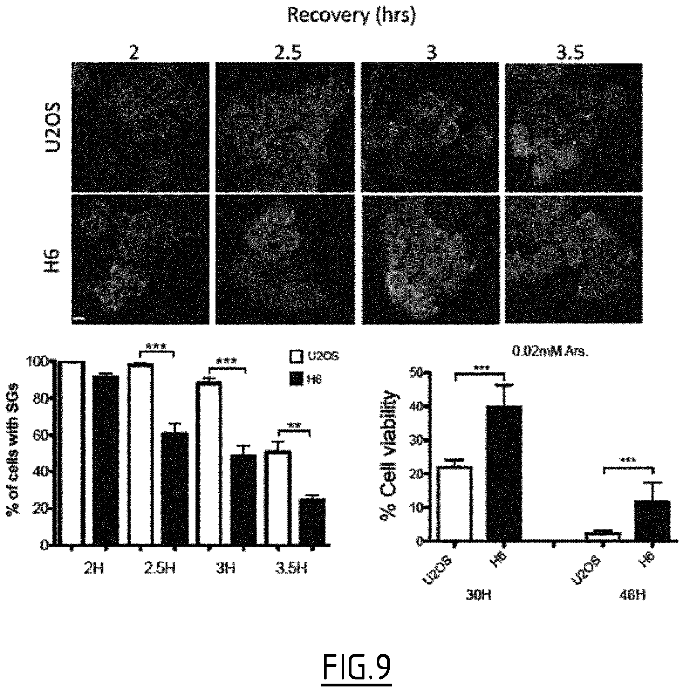

**Figure 9****:** Expression of Nb9 (AbR2DP46) (Nanobodies, top left-panel) accelerates SG elimination. Expression of Nb9 eliminates ALS-derived (TIA1-381T/E384K) aggregates in mouse-derived neurons (bottom panels).

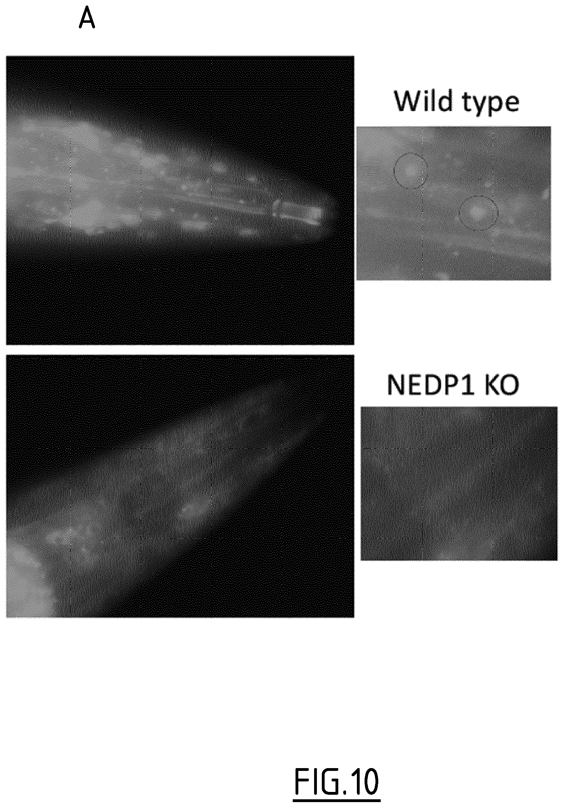

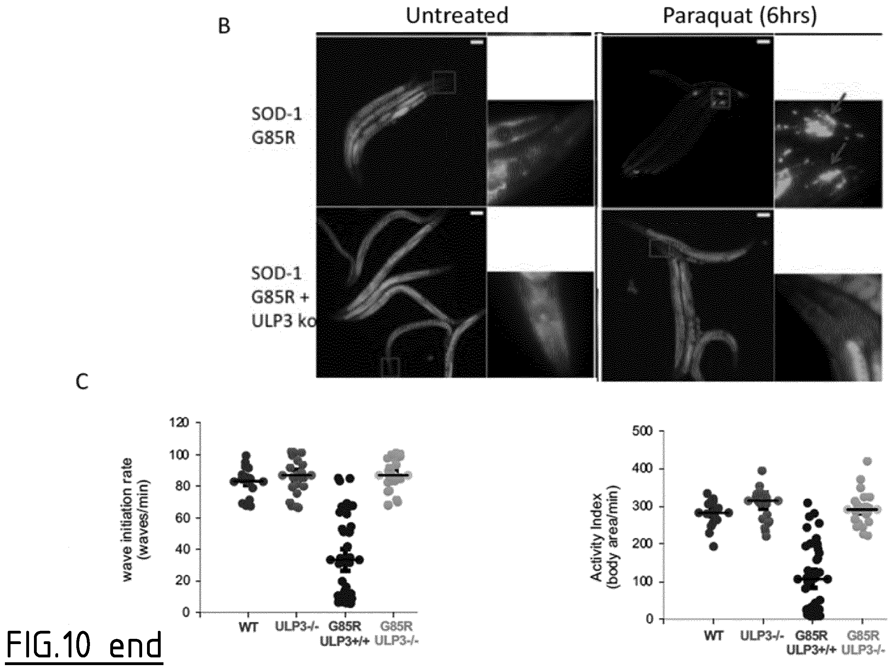

**Figure 10****:** A. NEDP1 deletion in *C. elegans* accelerates the elimination of SGs. Wild type and NEDP1 (Ulp-3) knockout C. elegans expressing GFP-G3BP1 (SG marker) were exposed to proteotoxic stress (Heat shock) and SGs were monitored by GFP live imaging. Red circles indicate SGs in the pharynx (containing neurons) of wild type animals. B. *C.elegans* expressing a SOD-1 ALS mutant (G85R) that causes the formation of aberrant SGs. Deletion of NEDP1 (Ulp-3) causes the elimination of aberrant SOD-1 mutant SGs. C. The SOD-1 ALS mutant dramatically decreases the mobility (wave initiation rate and activity index) of C. elegans but deletion of NEDP1 (Ulp-3) restores animal mobility almost to the wild type level.

### EXAMPLES

### Example 1

### Definitions

The following correspondence can be employed in the experimental part for single domain antibodies or nanobodies according to the invention:
- Nb1:; AbR2DP11
- Nb2: AbR2DP12 ;
- Nb3 : AbR2DP16;
- Nb4 :AbR2DP19 ;
- Nb5 :;AbR2DP23;
- Nb6: AbR2DP34;
- Nb7 : AbR2DP35;
- Nb8 AbR2DP44 ;
- Nb9: AbR2DP46;
- Nb10: AbR2DP47;
- Nb11: AbR2DP49;
- Nb12: AbR2DP55;
- Nb13: AbR2DP57;
- Nb14:AbR2DP58;
- Nb15AbR2DP61;
- Nb16:; AbR2DP62;
- Nb17: AbR2DP67;
- Nb18: AbR2DP69;
- Nb19: AbR2DP78;
- Nb20: AbR2DP80;
- Nb21 :AbR2DP93.

### Materials and Methods

### Reagents

All common chemicals were purchased from Sigma Aldrich. Protease Inhibitor Cocktail Tablets EDTA-free (Roche). Rabbit monoclonal anti-NEDD8 (1:2000), Y297 (GeneTex, GTX61205), rabbit anti-ubiquitin (1:2000), (DAKO, Z0458), mouse anti-GAPDH (1:5000) (6C5, ab8245), mouse anti-SUMO-2 (1:2000) (gift from Dr Guillaume Bossis), sheep anti-NEDP1 (1:1000) (gift from Prof. Ronald Hay), mouse anti-Flag (1:2000), (Sigma, F1804), mouse anti-His6 (Clontech, 631212). Anti-Flag beads were purchased from SIGMA.

### Cell culture

U2OS cells were grown in Dulbecco's modified Eagle's medium (DMEM) supplemented with 10 % fetal bovine serum (FBS) and antibiotics (Penicillin, 50 U/ml and Streptomycin 50 µg/ml) in 5 % CO₂ at 37)C in a humidified incubator. Cell lines were routinely tested for mycoplasma contamination but have not been authenticated. Cells were kept in culture for a maximum of 20 passages. Cells stably expressing Nbs were selected with G418 (1 mg/ml).

### Immunisation of Ilama, construction of anti-NEDP1 Nb library and selection of specific antibody fragments (Biopanning)

A llama was injected subcutaneously by 140 µg recombinant human NEDP1 protein emulsified with Gerbu adjuvant, six times at weekly intervals. After the last immunisation, 50 ml of blood were taken and peripheral blood lymphocytes (PBLs) were extracted with lympho-prep (Nycomed, Switzerland). Library construction was done as previously described (Saerens, D et al, S. Single-domain antibodies as building blocks for novel therapeutics. Curr. Opin. Pharmacol. 8, 600-608 (2008); Conrath, K. E. et al. -Lactamase Inhibitors Derived from Single-Domain Antibody Fragments Elicited in the Camelidae. Antimicrob. Agents Chemother. 45, 2807-2812 (2001)). Briefly, Trizolextracted mRNA from the pellet was used as a template for RT-PCR to generate first strand cDNA using oligo-dT primers cDNA was used as template to amplify 2 different products using specific primers for the variable domain until the CH2 domain CALL001 (SEQ ID N°106: 5'-GTCCTGGCTCTCTTCTACAAGG) and CALL002 (SEQ ID N°107: 5'-GGTACGTGCTGTTGAACTGTTCC) to amplify the heavy chain antibody gene fragments from the variable region to the CH2 region, which yielded the VH and the VHH containing gene fragments of 900 and 600 bp, respectively. The 600 bp product was excised from 1 % agarose gel, used as template for a second PCR via nested primers VHH-For primer 38 (SEQ ID N°108: 5'-GGA CTA GTG CGG CCG CTG GAG ACG GTG ACC TGG GT) and VHH-Back primer A6E (SEQ ID N°109: 5'-GAT GTG CAG CTG CAG GAG TCT GGR GGA GG) (Saerens, D., Ghassabeh, G. & Muyldermans, S. Single-domain antibodies as building blocks for novel therapeutics. Curr. Opin. Pharmacol. 8, 600-608 (2008)) to amplify the VHH sequence of about 400 bp and digested with Not I and Pstl restriction enzymes (NEB). The PCR fragments were ligated into the phagemid vector pHEN4 and transformed into electro-competent E. *coli* TG1 cells. The resulting Nb library was infected with M13K07 helper phages for the expression of Nbs on the phages. Biopanning was performed by coating ELISA plates (Nunc) with 20 µg of recombinant NEDP1 per/well. Phage enrichment was achieved by 3 rounds of *in vitro* selection.

Phages were eluted as described (Conrath, K. E. et al. -Lactamase Inhibitors Derived from Single-Domain Antibody Fragments Elicited in the Camelidae. Antimicrob. Agents Chemother. 45, 2807-2812 (2001)). TG1 *E.coli* cells were infected with the eluted phages, and selected from LB ampicillin plates. One hundred viable colonies were randomly picked from each round of panning and their VHH was expressed as described (Conrath, K. E. et al. -Lactamase Inhibitors Derived from Single-Domain Antibody Fragments Elicited in the Camelidae. Antimicrob. Agents Chemother. 45, 2807-2812 (2001)). The periplasmic extracts (PE) were obtained through osmotic shock as described (Skerra, A. & Pluckthun, A. Assembly of a functional immunoglobulin Fv fragment in Escherichia coli. Science 240, 1038-1041 (1988)). The enrichment of each round of panning was checked by a polyclonal phage ELISA with anti-M13-HRP antibodies.

### Expression and purification of antibody fragments for ELISA testing

The cloned insert expressing Nbs recognizing NEDP1 recombinant protein, was sequenced using the RP or GIII primer and sequences were grouped based on the differences in their complementarity determining regions (CDRs). Representatives of each group were recloned into the expression vector pHEN6 using BstEII and Pstl (NEB) enzymes. The plasmid constructs were transformed into WK6 *E. coli* cells and large quantities of His6 tagged recombinant Nbs were purified with 1 ml of HIS-Select Nickel Affinity Gel (Sigma-Aldrich) following periplasmic expression through osmotic shock. Elution was performed with 1 column volume of 0.5 M imidazole, repeated 3 times. The Nb yields after purification varied from 1 to 15 mg/L of *E. coli* culture. Solid phase ELISA was performed by coating recombinant NEDP1 (20 µg/well) on Maxisorb 96 well plates (Nunc) at 4 °C overnight. The ELISA was performed using purified Nbs as primary antibodies, followed by an anti-His6 and an anti-mouse antibody conjugated to horseradish peroxidase (Sigma-Aldrich). A non-relevant Nb (NbAahl)36 was used as negative control for antibody detection while a non-relevant protein, Bovine Serum Albumin (BSA) was used as negative control for NEDP1 coating.

### Cross-reactivity assay

The specificity of the 17 purified Nbs was tested by indirect ELISA, and 3 different proteins, NEDP1, ULP3 (the *C. elegans* homologue of NEDP1) and GST were chosen to test potential cross-reactivity. The same amount of the 3 proteins (20 µg) was used to coat a 96-well plate, incubated overnight at 4°C. After blocking, purified Nbs were incubated with antigen at 37 °C for 1h. Then, anti-His6 (diluted 1:2000) was added and incubated at 37°C for 1h. After another washing step, an anti-mouse antibody conjugated to horseradish peroxidase (Sigma-Aldrich) was added to the plate and incubated at 37 °C for 1h. The reaction was stopped by the addition of 50 µl of 3 M H₂SO₄, and then the absorbance at 492 nm was read on a microtiter plate reader.

The specificity of anti-NEDP1 Nbs was evaluated by monitoring their interaction against recombinant human NEDP1, ULP-3 and GST as GST-NEDP1 was used for immunisation. Only candidates with no cross reactivity to ULP-3 and GST, were selected to measure their affinity by surface plasmon resonance.

### Surface Plasmon Resonance (SPR) analysis

Nbs affinities for NEDP1 were determined on a Biacore 2000 apparatus (Biacore AB, Uppsala, Sweden). Nanobodies (355nM) were immobilized on anti-His6 antibody covalently immobilized on a carboxymethyl dextran sensorchip (CM5 Biacore AB) using the amine coupling method according to the manufacturer's instructions. The immobilization level was 500 resonance units (RU). Different concentrations of NEDP1 ranging from 0.75 to 200 nM in HEPES-buffered saline (0.01 M HEPES, pH 7.4, 0.15 M NaCl, 3 mM EDTA, 0.005 % surfactant P20) were injected and exposed to the Nb. All sensorgrams were fitted by subtracting the signal from the reference flow cell and were treated using BIA evaluation version 3.2 software (Biacore AB).

### Expression and Purification of Recombinant Proteins

His6-MBP-NEDD8-Ub and GST-NEDP1 (wild type and catalytic C163A mutant) were expressed and purified from, *Escherichia coli* BL21 (DE3) as previously described (Shen, L. et al. Structural basis of NEDD8 ubiquitin discrimination by the deNEDDylating enzyme NEDP1. EMBO J. 24, 1341-1351 (2005); Mendoza, H. M. et al. NEDP1, a highly conserved cysteine protease that deNEDDylates Cullins. J. Biol. Chem. 278, 25637-25643 (2003)). Expression of the protein was confirmed by western blotting, and the activity of recombinant protein was tested by in vitro deconjugation and processing assays.

### In vitro deconjugation assays

To determine the deconjugation activity of NEDP1, an increasing amount of the recombinant enzyme was incubated with 20 µg of extracts from U2OS NEDP1 knockout cells (H6) that have high levels of NEDD8 conjugates (Bailly, A. P. et al. The Balance between Mono- and NEDD8-Chains Controlled by NEDP1 upon DNA Damage Is a Regulatory Module of the HSP70 ATPase Activity. Cell Rep. 29, 212-224.e8 (2019)) at 37 °C for 30 min in 15µl of reaction buffer containing 50 mM Tris-HCI (pH 8.0), 50 mM NaCl and 5 mM b-mercaptoethanol. The reaction was stopped by the addition of 3 µl of 6X SDS sample buffer and analysed by SDS-PAGE and Coomassie blue staining.

### In vitro processing assays

The His6-MBP-NEDD8-Ub was expressed using vector pLous3 and purified using Ni-NTA-agarose Shen, L. et al. Structural basis of NEDD8 ubiquitin discrimination by the deNEDDylating enzyme NEDP1 (EMBO J. 24, 1341-1351 (2005)). 600 ng of His6-MBP-NEDD8-Ub substrate was incubated with recombinant human NEDP1 protein at 37 °C for 30 min in 15 µl of reaction buffer containing 50 mM Tris-HCI (pH 8.0), 50 mM NaCl and 5 mM b-mercaptoethanol. The reaction was stopped by the addition of 3 µl of 6X SDS sample buffer and analysed by SDS-PAGE and Coomassie blue staining (Shen, L. et al. Structural basis of NEDD8 ubiquitin discrimination by the deNEDDylating enzyme NEDP1. EMBO J. 24, 1341-1351 (2005)). To test the NEDP1 processing inhibitory function of the nanobodies, 600 ng of His6-MBP-NEDD8-Ub was incubated with 300 ng of NEDP1 and increasing amount of the Nbs.

### Immunoprecipitations

U2OS cells either with empty pcDNA3 vector or stably expressing Nb2/Nb9-Flag constructs were lysed in 50 mM Tris-HCI, pH 7.4, 100 mM KCI, 1 % NP-40, 2 mM EDTA, protease inhibitors (complete EDTA-free, Roche) for 15 min on ice. After centrifugation at 13000 rpm for 15 min at 4°C extracts were used for immunoprecipitations overnight at 4°C with 20 µl of pre-washed Flag beads. Next day beads were washed 3x with 500 µl of lysis buffer, resuspended in 50 µl of 2xSDS Laemmli buffer and boiled for 5 min. Eluates were analysed by western blotting.

### Western blot analysis

For all inputs we routinely used approximately 20 µg of total protein. Proteins were resolved in 4-12% precast Bis-Tris gels and transferred onto PVDF membrane using the Bio-Rad Mini Trans-Blot apparatus. Membranes were blocked in 5 % milk solution (PBS with 0.1 % Tween-20 and 5 % skimmed milk) for 1 h at room temperature with gentle agitation. Membranes were incubated with the primary antibodies diluted in PBS, 0.1 % Tween-20, 3 % BSA and 0.1 % NaN3, overnight at 4°C. Membranes were washed 3x10min with PBS, 0.1 % Tween-20 and then incubated with the appropriate secondary antibody (1:2000) (Sigma Aldrich) for 1 h at room temperature (5 % milk). After incubation, membranes were washed 2x15min with PBS, 0.1 % Tween-20 followed by 2x5 min with PBS. Detection was performed with ECL Western Blotting Detection Reagents and membranes were exposed to X-ray Medical Film before being developed.

### Results

### Selection of NEDP1 Nbs

An immune response against recombinant NEDP1 was elicited in llama. The peripheral blood lymphocytes of the llama were used to clone the VHH repertoire in a phage display vector. A VHH library of about 3 × 10⁸ independent transformants was obtained. A colony PCR on 20 clones from the library indicated that 93% of transformants harbored the vector with the right insert size for a VHH gene. Representative aliquots of the library were rescued with M13K07 helper phages, and the highest enrichment was obtained after the third round of panning (100-fold). Finally, based on sequence data, 21 different Nbs (Fig. 1A) were identified. The specificity of the isolated Nbs against human NEDP1 and ULP-3 and was also determined by ELISA as described in Materials and Methods (Fig. 1A). Further ELISA testing with increasing amounts of coated recombinant NEDP1 confirmed the interaction of a series of isolated Nbs with NEDP1 (Fig. 1B). The affinities of selected Nbs against NEDP1 were determined by surface plasmon resonance as described in Materials and Methods. The Nbs were immobilized on CM5 sensor chips and dilutions of NEDP1 were in the mobile phase to record the association kinetics. The binding kinetics between the Nbs and NEDP1 yielded KDs values that vary from 6,98 10-9 M to <10-11 M (Fig. 1C). Nbs with high affinities, purification yields and specificity for human NEDP1 (AbR2DP12, AbR2DP46 and AbR2DP47) were selected for further functional validation.

Anti-NEDP1 Nbs inhibit both the processing and deconjugating activity of NEDP1 in vitro NEDP1 was originally discovered as a specific NEDD8 processing enzyme exposing the diGly motif at the C-terminus of NEDD8 but it also promotes deconjugation of NEDD8 from substrates (Abidi, N. & Xirodimas, D. P. Regulation of cancer-related pathways by protein NEDDylation and strategies for the use of NEDD8 inhibitors in the clinic. Endocr. Relat. Cancer 22, T55-T70 (2015); Enchev, R. I., Schulman, B. A. & Peter, M. Protein neddylation: beyond cullin-RING ligases. Nat. Rev. Mol. Cell Biol. 16, 30-44 (2014)). We first determined the effect of the Nbs on the processing activity of NEDP1 in vitro. The assay is based on monitoring the activity of NEDP1 to cleave the His6-MBP-NEDD8-ubiquitin fusion. The release of ubiquitin is indicative of the processing activity of NEDP1 (Fig. 2A). As expected wild type NEDP1 can process *in vitro* the His6-MBP-NEDD8-ubiquitin fusion (Fig. 2B). Addition of increasing amounts of purified AbR2DP46, completely inhibited NEDP1 processing activity at stoichiometric amounts (Fig. 2B). Similar results were obtained with AbR2DP12 and AbR2DP47 (Fig. 2C). In contrast, Nb7 which also binds to NEDP1 with high affinity (Fig. 1C), had no effect on the NEDP1 processing activity, strongly indicating that the inhibitory effect of Nbs 2, 9, 10 is specific and not due to a co-purified bacterial contaminating activity.

We next evaluated the effect of the developed Nbs on the deconjugating activity of NEDP1 (Fig. 3A). For this, we used extracts from NEDP1 knockout U2OS cells (H6) that have high levels of mono- and poly-NEDDylated conjugates19. Addition of wild type but not catalytically inactive recombinant NEDP1 (Fig. 3B, 3C) dramatically reduces NEDDylation in the extracts indicative of the deconjugating activity of NEDP1. Similarly to the processing assay, addition of purified Nbs AbR2DP12, AbR2DP46 and AbR2DP47 at stoichiometric amounts to the recombinant NEDP1, caused a complete inhibition in the deconjugating activity of NEDP1 (Fig. 3C).

### Anti-NEDP1 Nbs inhibit NEDP1 activity in vivo

To assess the effect of the developed Nbs on the activity of NEDP1 *in vivo* we developed stable U2OS osteosarcoma cell lines expressing Flag-tag versions of Nbs. By anti-Flag immunoprecipitations we found that the expressed Nbs interact with endogenous NEDP1 (Fig. 4A). Consistent with an inhibitory role of the Nbs on NEDP1 function, the levels of NEDDylation are dramatically increased in cells expressing the Nbs, compared to control cells (empty pcDNA3 vector) (Fig. 4A). No effect on the levels of NEDP1 was observed, indicating that the Nbs affect the catalytic activity but not the protein levels of NEDP1 (Fig. 4A). Importantly, both the profile and levels of NEDDylation in cells expressing the Nbs is very similar to those observed in NEDP1 knockout cells (Bailly, A. P. et al. The Balance between Mono- and NEDD8-Chains Controlled by NEDP1 upon DNA Damage Is a Regulatory Module of the HSP70 ATPase Activity. Cell Rep. 29, 212-224.e8 (2019)), especially on the depletion of free NEDD8, strongly suggesting that the Nbs are potent inhibitors of NEDP1 in cells. No effect on ubiquitination or SUMO-2 conjugation was observed, indicating the specificity of the Nbs towards NEDP1 and the NEDD8 pathway (Fig. 4B). The combination of the above experiments show that the developed Nbs are potent inhibitors of both the processing and deconjugating activity both *in vitro* and *in vivo.*

### Example 2

Amyotrophic Lateral Sclerosis (ALS) is currently an incurable motor neuron disease that originates due to the generation of toxic protein aggregates. Developing approaches that would eliminate the toxic aggregates is regarded as a therapeutic approach and it is at the forefront of research and therapeutic intervention. We found that inhibition of an enzyme called NEDP1, causes the elimination of such toxic aggregates *in vitro* (tissue culture cell lines and mouse neurons) and in model organisms (*C. elegans*) and improves cell survival. We used the Nbs according to the invention as the first available potent inhibitors for NEDP1, representing research tools that can be commercialized. We found that the NEDP1 Nbs are very efficient *in vitro* to eliminate toxic aggregates that appear in ALS patients. Our studies have revealed NEDP1 as potential therapeutic target for ALS and the developed Nbs as potential therapeutic tools.

### Accumulation of toxic aggregates as cause for ALS pathology

Amyotrophic Lateral Sclerosis (ALS) is a motor neuron disease representing a well-established example of how defects in the elimination of protein aggregates leads to neurodegeneration. In normal cells, exposure to proteotoxic stress (heat shock or oxidative stress) leads to the formation of cytoplasmic protein inclusions called stress granules (SGs) that slow-down protein synthesis during stress. Once stress is alleviated, SGs are disassembled, protein synthesis is resumed indicative of the recovery process (Ivanov P, Kedersha N, Anderson P. Stress Granules and Processing Bodies in Translational Control. Cold Spring Harb Perspect Biol 2019; 11: a032813; Wheeler JR, Matheny T, Jain S, Abrisch R, Parker R. Distinct stages in stress granule assembly and disassembly. eLife 2016; 5: e18413) (Fig. 5). However, in ALS the above process is severely compromised, as mutations in genes encoding for SGs proteins result in the formation of aberrant SGs aggregates. In contrast to the physiological response, aberrant SGs persist in the cytoplasm (Fig. 5, 6) and cause toxicity. The disease is characterized by the degeneration specifically of motor neurons, which eventually leads to muscle paralysis. Consequently, it is believed that the elimination of such aberrant aggregates would permit reversal of such symptoms (Advani VM, Ivanov P. Stress granule subtypes: an emerging link to neurodegeneration. Cell Mol Life Sci 2020; 77: 4827-4845). Defining approaches that would promote the elimination of toxic inclusions in motor neurons of ALS patients is at the forefront of the scientific research and a major therapeutic challenge for pharmaceutical companies. This is particularly critical for ALS patients, as currently there is no treatment or anything that improves life-expectancy (usually 2-5 years post diagnosis) or quality of life (Advani VM, Ivanov P. Stress granule subtypes: an emerging link to neurodegeneration. Cell Mol Life Sci 2020; 77: 4827-4845).

### The Ubiguitin/Ubiguitin-like system and protein aggregation

One of the main effectors and regulators of protein aggregation is the family of Ubiquitin and Ubiquitin-like molecules (Ubls) including SUMO and NEDD8. These small proteins are covalently attached on substrates as post-translational modifications through the activity of three enzymes named E1, E2 and E3 (Abidi N, Xirodimas DP. Regulation of cancer-related pathways by protein NEDDylation and strategies for the use of NEDD8 inhibitors in the clinic. Endocrine-Related Cancer 2015; 22: T55-T70). The process is highly dynamic and the extent of substrate modification is finely balanced by the action of deconjugating enzymes that remove Ubiquitin/Ubls from substrates (Fig. 7) (Abidi N, Xirodimas DP. Regulation of cancer-related pathways by protein NEDDylation and strategies for the use of NEDD8 inhibitors in the clinic. Endocrine-Related Cancer 2015; 22: T55-T70).

### The NEDD8 pathway as regulator of SGs - NEDP1 as therapeutic target for ALS

We revealed that NEDP1, the enzyme that produces the mature form of NEDD8 (processing activity) and also deconjugates NEDD8 from proteins (deconjugating activity) (Fig. 7, 2, 3) is directly involved in the dynamics of SG elimination. Knockout of NEDP1 in human cells but also in a model organism such as *C. elegans,* accelerates the elimination of SGs induced upon proteotoxic stress (arsenic) and improves cell survival.

### Activity of Nanobodies according to the invention

Prior to its activation by the E1 enzyme, NEDD8 has to be processed in the C-terminus to expose the diglycine motif that is required for activation. NEDP1 is the processing enzyme that performs this function. In addition, NEDP1 has also deconjugating activity that removes NEDD8 from substrates (Fig. 7, 2, 3). We developed Nbs as potent inhibitors for NEDP1, that block both the processing (Fig. 2) and deconjugating activity of the enzyme (Fig. 3). We found that NEDP1 gene deletion (CRISPR/Cas9) in human tissue culture cell lines (U2OS osteosarcoma) accelerates the elimination of SGs induced upon stress (arsenic) (Fig. 8). This is correlated to improved cell survival (Fig. 8). Critically, we found that expression of the developed Nbs AbR2DP46 also accelerates the elimination of toxic aggregates found in ALS, in mouse derived neuron cells (hippocampal) (Fig. 9). The *in vitro* studies were also confirmed in vivo using *C. elegans* as model organism. Similarly to what we observe in human cells, deletion of Ulp-3 (NEDP1 homologue in *C. elegans)* causes almost complete disappearance of SGs induced upon stress (Fig. 11A). Additionally, we used a C. elegans strain that expresses at endogenous levels the SOD-1 ALS mutant G85R. This mutation is often seen in ALS patients in humans and generates the formation of aberrant SGs. Indeed, exposure of SOD-1^{G85R} C. elegans to paraquat (proteotoxic stress) induces the formation of aberrant SGs. However, deletion of NEDP1 (Ulp-3) almost completely prevents the formation of aberrant SGs. Critically, we also perform an assay that monitors the mobility of C. elegans. The SOD-1^{G85R} mutation causes a dramatic decrease in animal mobility as measured by the wave initiation rate and activity index (parameters of animal mobility) (Fig. 11C). The SOD-1^{G85R} ALS mutation causes degeneration of motor neurons and subsequently animal muscle paralysis, which recapitulates what is observed in human ALS patients. Deletion of NEDP1 (Ulp-3) almost completely rescues the SOD-1^{G85R}

## Claims

1. A kit for inhibiting the deNEDDylating enzyme NEDP1, in particular for the prevention and/or treatment of neurodegenerative diseases such as Amyotrophic Lateral Sclerosis and/or Frontotemporal Degeneration, said kit comprising at least one isolated single domain antibody specific for the deNEDDylating enzyme NEDP1 chosen from:
- an isolated single domain antibody called AbR2DP44 which comprises CDR-H1 of sequence SEQ ID NO:1, CDR-H2 of sequence SEQ ID NO:2 and CDR-H3 of sequence SEQ ID NO:3;
- an isolated single domain antibody called AbR2DP58 which comprises CDR-H1 of sequence SEQ ID NO:4, CDR-H2 of sequence SEQ ID NO:5 and CDR-H3 of sequence SEQ ID NO:6;
- an isolated single domain antibody called AbR2DP61 which comprises CDR-H1 of sequence SEQ ID NO:7, CDR-H2 of sequence SEQ ID NO:8 and CDR-H3 of sequence SEQ ID NO:9;
- an isolated single domain antibody called AbR2DP80 which comprises CDR-H1 of sequence SEQ ID NO:10, CDR-H2 of sequence SEQ ID NO:11 and CDR-H3 of sequence SEQ ID NO:12;
- an isolated single domain antibody called AbR2DP16 which comprises CDR-H1 of sequence SEQ ID NO:13, CDR-H2 of sequence SEQ ID NO:14 and CDR-H3 of sequence SEQ ID NO:15;
- an isolated single domain antibody called AbR2DP23 which comprises CDR-H1 of sequence SEQ ID NO:16, CDR-H2 of sequence SEQ ID NO:17 and CDR-H3 of sequence SEQ ID NO:18;
- an isolated single domain antibody called AbR2DP47 which comprises CDR-H1 of sequence SEQ ID NO:19, CDR-H2 of sequence SEQ ID NO:20 and CDR-H3 of sequence SEQ ID NO:21;
- an isolated single domain antibody called AbR2DP49 which comprises CDR-H1 of sequence SEQ ID NO:22, CDR-H2 of sequence SEQ ID NO:23 and CDR-H3 of sequence SEQ ID NO:24;
- an isolated single domain antibody called AbR2DP69 which comprises CDR-H1 of sequence SEQ ID NO:25, CDR-H2 of sequence SEQ ID NO:26 and CDR-H3 of sequence SEQ ID NO:27;
- an isolated single domain antibody called AbR2DP19 which comprises CDR-H1 of sequence SEQ ID NO:28, CDR-H2 of sequence SEQ ID NO:29 and CDR-H3 of sequence SEQ ID NO:30;
- an isolated single domain antibody called AbR2DP93 which comprises CDR-H1 of sequence SEQ ID NO:31, CDR-H2 of sequence SEQ ID NO:32 and CDR-H3 of sequence SEQ ID NO:33;
- an isolated single domain antibody called AbR2DP11 which comprises CDR-H1 of sequence SEQ ID NO:34, CDR-H2 of sequence SEQ ID NO:35 and CDR-H3 of sequence SEQ ID NO:36;
- an isolated single domain antibody called AbR2DP46 which comprises CDR-H1 of sequence SEQ ID NO:37, CDR-H2 of sequence SEQ ID NO:38 and CDR-H3 of sequence SEQ ID NO:39;
- an isolated single domain antibody called AbR2DP67 which comprises CDR-H1 of sequence SEQ ID NO:40, CDR-H2 of sequence SEQ ID NO:41 and CDR-H3 of sequence SEQ ID NO:42;
- an isolated single domain antibody called AbR2DP34 which comprises CDR-H1 of sequence SEQ ID NO:43, CDR-H2 of sequence SEQ ID NO:44 and CDR-H3 of sequence SEQ ID NO:45;
- an isolated single domain antibody called AbR2DP78 which comprises CDR-H1 of sequence SEQ ID NO:46, CDR-H2 of sequence SEQ ID NO:47 and CDR-H3 of sequence SEQ ID NO:48;
- an isolated single domain antibody called AbR2DP12 which comprises CDR-H1 of sequence SEQ ID NO:49, CDR-H2 of sequence SEQ ID NO:50 and CDR-H3 of sequence SEQ ID NO:51;
- an isolated single domain antibody called AbR2DP57 which comprises CDR-H1 of sequence SEQ ID NO:52, CDR-H2 of sequence SEQ ID NO:53 and CDR-H3 of sequence SEQ ID NO:54;
- an isolated single domain antibody called AbR2DP62 which comprises CDR-H1 of sequence SEQ ID NO:55, CDR-H2 of sequence SEQ ID NO:56 and CDR-H3 of sequence SEQ ID NO:57; and
- an isolated single domain antibody called AbR2DP55 which comprises CDR-H1 of sequence SEQ ID NO:61, CDR-H2 of sequence SEQ ID NO:62 and CDR-H3 of sequence SEQ ID NO:63.

2. The kit according to claim 1, wherein said at least one isolated single domain antibody specific for the deNEDDylating enzyme NEDP1 is chosen from:
- an isolated single domain antibody called AbR2DP44 having the sequence set forth as SEQ ID NO:64 or a sequence at least 85% identical to SEQ ID NO:64;
- an isolated single domain antibody called AbR2DP58 having the sequence set forth as SEQ ID NO:65 or a sequence at least 85% identical to SEQ ID NO:65;
- an isolated single domain antibody called AbR2DP61 having the sequence set forth as SEQ ID NO:66 or a sequence at least 85% identical to SEQ ID NO:66;
- an isolated single domain antibody called AbR2DP80 having the sequence set forth as SEQ ID NO:67 or a sequence at least 85% identical to SEQ ID NO:67;
- an isolated single domain antibody called AbR2DP16 having the sequence set forth as SEQ ID NO:68 or a sequence at least 85% identical to SEQ ID NO:68;
- an isolated single domain antibody called AbR2DP23 having the sequence set forth as SEQ ID NO:69 or a sequence at least 85% identical to SEQ ID NO:69;
- an isolated single domain antibody called AbR2DP47 having the sequence set forth as SEQ ID NO:70 or a sequence at least 85% identical to SEQ ID NO:70;
- an isolated single domain antibody called AbR2DP49 having the sequence set forth as SEQ ID NO:71 or a sequence at least 85% identical to SEQ ID NO:71;
- an isolated single domain antibody called AbR2DP69 having the sequence set forth as SEQ ID NO:72 or a sequence at least 85% identical to SEQ ID NO:72;
- an isolated single domain antibody called AbR2DP19 having the sequence set forth as SEQ ID NO:73 or a sequence at least 85% identical to SEQ ID NO:73;
- an isolated single domain antibody called AbR2DP93 having the sequence set forth as SEQ ID NO:74 or a sequence at least 85% identical to SEQ ID NO:74;
- an isolated single domain antibody called AbR2DP11 having the sequence set forth as SEQ ID NO:75 or a sequence at least 85% identical to SEQ ID NO:75;
- an isolated single domain antibody called AbR2DP46 having the sequence set forth as SEQ ID NO:76 or a sequence at least 85% identical to SEQ ID NO:76;
- an isolated single domain antibody called AbR2DP67 having the sequence set forth as SEQ ID NO:77 or a sequence at least 85% identical to SEQ ID NO:77;
- an isolated single domain antibody called AbR2DP34 having the sequence set forth as SEQ ID NO:78 or a sequence at least 85% identical to SEQ ID NO:78;
- an isolated single domain antibody called AbR2DP78 having the sequence set forth as SEQ ID NO:79 or a sequence at least 85% identical to SEQ ID NO:79;
- an isolated single domain antibody having the sequence set forth as SEQ ID NO:80 or a sequence at least 85% identical to SEQ ID NO:80;
- an isolated single domain antibody called AbR2DP57 having the sequence set forth as SEQ ID NO:81 or a sequence at least 85% identical to SEQ ID NO:81;
- an isolated single domain antibody called AbR2DP62 having the sequence set forth as SEQ ID NO:82 or a sequence at least 85% identical to SEQ ID NO:82; and
- an isolated single domain antibody called AbR2DP55 having the sequence set forth as SEQ ID NO:84 or a sequence at least 85% identical to SEQ ID NO:84.

3. At least one isolated single domain antibody chosen from:
- an isolated single domain antibody called AbR2DP44 which comprises CDR-H1 of sequence SEQ ID NO:1, CDR-H2 of sequence SEQ ID NO:2 and CDR-H3 of sequence SEQ ID NO:3;
- an isolated single domain antibody called AbR2DP58 which comprises CDR-H1 of sequence SEQ ID NO:4, CDR-H2 of sequence SEQ ID NO:5 and CDR-H3 of sequence SEQ ID NO:6;
- an isolated single domain antibody called AbR2DP61 which comprises CDR-H1 of sequence SEQ ID NO:7, CDR-H2 of sequence SEQ ID NO:8 and CDR-H3 of sequence SEQ ID NO:9;
- an isolated single domain antibody called AbR2DP80 which comprises CDR-H1 of sequence SEQ ID NO:10, CDR-H2 of sequence SEQ ID NO:11 and CDR-H3 of sequence SEQ ID NO:12;
- an isolated single domain antibody called AbR2DP16 which comprises CDR-H1 of sequence SEQ ID NO:13, CDR-H2 of sequence SEQ ID NO:14 and CDR-H3 of sequence SEQ ID NO:15;
- an isolated single domain antibody called AbR2DP23 which comprises CDR-H1 of sequence SEQ ID NO:16, CDR-H2 of sequence SEQ ID NO:17 and CDR-H3 of sequence SEQ ID NO:18;
- an isolated single domain antibody called AbR2DP47 which comprises CDR-H1 of sequence SEQ ID NO:19, CDR-H2 of sequence SEQ ID NO:20 and CDR-H3 of sequence SEQ ID NO:21;
- an isolated single domain antibody called AbR2DP49 which comprises CDR-H1 of sequence SEQ ID NO:22, CDR-H2 of sequence SEQ ID NO:23 and CDR-H3 of sequence SEQ ID NO:24;
- an isolated single domain antibody called AbR2DP69 which comprises CDR-H1 of sequence SEQ ID NO:25, CDR-H2 of sequence SEQ ID NO:26 and CDR-H3 of sequence SEQ ID NO:27;
- an isolated single domain antibody called AbR2DP19 which comprises CDR-H1 of sequence SEQ ID NO:28, CDR-H2 of sequence SEQ ID NO:29 and CDR-H3 of sequence SEQ ID NO:30;
- an isolated single domain antibody called AbR2DP93 which comprises CDR-H1 of sequence SEQ ID NO:31, CDR-H2 of sequence SEQ ID NO:32 and CDR-H3 of sequence SEQ ID NO:33;
- an isolated single domain antibody called AbR2DP11 which comprises CDR-H1 of sequence SEQ ID NO:34, CDR-H2 of sequence SEQ ID NO:35 and CDR-H3 of sequence SEQ ID NO:36;
- an isolated single domain antibody called AbR2DP46 which comprises CDR-H1 of sequence SEQ ID NO:37, CDR-H2 of sequence SEQ ID NO:38 and CDR-H3 of sequence SEQ ID NO:39;
- an isolated single domain antibody called AbR2DP67 which comprises CDR-H1 of sequence SEQ ID NO:40, CDR-H2 of sequence SEQ ID NO:41 and CDR-H3 of sequence SEQ ID NO:42;
- an isolated single domain antibody called AbR2DP34 which comprises CDR-H1 of sequence SEQ ID NO:43, CDR-H2 of sequence SEQ ID NO:44 and CDR-H3 of sequence SEQ ID NO:45;
- an isolated single domain antibody called AbR2DP78 which comprises CDR-H1 of sequence SEQ ID NO:46, CDR-H2 of sequence SEQ ID NO:47 and CDR-H3 of sequence SEQ ID NO:48;
- an isolated single domain antibody called AbR2DP12 which comprises CDR-H1 of sequence SEQ ID NO:49, CDR-H2 of sequence SEQ ID NO:50 and CDR-H3 of sequence SEQ ID NO:51;
- an isolated single domain antibody called AbR2DP57 which comprises CDR-H1 of sequence SEQ ID NO:52, CDR-H2 of sequence SEQ ID NO:53 and CDR-H3 of sequence SEQ ID NO:54;
- an isolated single domain antibody called AbR2DP62 which comprises CDR-H1 of sequence SEQ ID NO:55, CDR-H2 of sequence SEQ ID NO:56 and CDR-H3 of sequence SEQ ID NO:57; and
- an isolated single domain antibody called AbR2DP55 which comprises CDR-H1 of sequence SEQ ID NO:61, CDR-H2 of sequence SEQ ID NO:62 and CDR-H3 of sequence SEQ ID NO:63.
for use for inhibiting the deNEDDylating enzyme NEDP1.

4. At least one isolated single domain antibody for use according to claim 3, wherein said at least one isolated single domain antibody is chosen from:
- an isolated single domain antibody called AbR2DP44 having the sequence set forth as SEQ ID NO:64 or a sequence at least 85% identical to SEQ ID NO:64;
- an isolated single domain antibody called AbR2DP58 having the sequence set forth as SEQ ID NO:65 or a sequence at least 85% identical to SEQ ID NO:65;
- an isolated single domain antibody called AbR2DP61 having the sequence set forth as SEQ ID NO:66 or a sequence at least 85% identical to SEQ ID NO:66;
- an isolated single domain antibody called AbR2DP80 having the sequence set forth as SEQ ID NO:67 or a sequence at least 85% identical to SEQ ID NO:67;
- an isolated single domain antibody called AbR2DP16 having the sequence set forth as SEQ ID NO:68 or a sequence at least 85% identical to SEQ ID NO:68;
- an isolated single domain antibody called AbR2DP23 having the sequence set forth as SEQ ID NO:69 or a sequence at least 85% identical to SEQ ID NO:69;
- an isolated single domain antibody called AbR2DP47 having the sequence set forth as SEQ ID NO:70 or a sequence at least 85% identical to SEQ ID NO:70;
- an isolated single domain antibody called AbR2DP49 having the sequence set forth as SEQ ID NO:71 or a sequence at least 85% identical to SEQ ID NO:71;
- an isolated single domain antibody called AbR2DP69 having the sequence set forth as SEQ ID NO:72 or a sequence at least 85% identical to SEQ ID NO:72;
- an isolated single domain antibody called AbR2DP19 having the sequence set forth as SEQ ID NO:73 or a sequence at least 85% identical to SEQ ID NO:73;
- an isolated single domain antibody called AvR2DP93 having the sequence set forth as SEQ ID NO:74 or a sequence at least 85% identical to SEQ ID NO:74;
- an isolated single domain antibody called AbR2DP11 having the sequence set forth as SEQ ID NO:75 or a sequence at least 85% identical to SEQ ID NO:75;
- an isolated single domain antibody called AbR2DP46 having the sequence set forth as SEQ ID NO:76 or a sequence at least 85% identical to SEQ ID NO:76;
- an isolated single domain antibody called AbR2DP67 having the sequence set forth as SEQ ID NO:77 or a sequence at least 85% identical to SEQ ID NO:77;
- an isolated single domain antibody called AbR2DP34 having the sequence set forth as SEQ ID NO:78 or a sequence at least 85% identical to SEQ ID NO:78;
- an isolated single domain antibody called AbR2DP78 having the sequence set forth as SEQ ID NO:79 or a sequence at least 85% identical to SEQ ID NO:79;
- an isolated single domain antibody called AbR2DP12 having the sequence set forth as SEQ ID NO:80 or a sequence at least 85% identical to SEQ ID NO:80;
- an isolated single domain antibody called AbR2DP57 having the sequence set forth as SEQ ID NO:81 or a sequence at least 85% identical to SEQ ID NO:81;
- an isolated single domain antibody called AbR2DP62 having the sequence set forth as SEQ ID NO:82 or a sequence at least 85% identical to SEQ ID NO:82; and
- an isolated single domain antibody called AbR2DP55 having the sequence set forth as SEQ ID NO:84 or a sequence at least 85% identical to SEQ ID NO:84.

5. At least one isolated single domain antibody for use according to claim 3 or 4 to inhibit the activity of NEDP1 *in vitro* reactions, in cell extracts and in live cells.

6. At least one isolated single domain antibody for use according to any of claims 3 to 5 for the prevention and/or treatment of neurodegenerative diseases.

7. At least one isolated single domain antibody for use according to claim 6, wherein said neurodegenerative disease is chosen from Amyotrophic Lateral Sclerosis, Frontotemporal Degeneration, Huntington's disease and Alzheimer's disease.

8. At least one isolated single domain antibody for use according to any of claims 3 to 7, wherein said at least one isolated single domain antibody is comprised in a pharmaceutical composition.

9. The kit according to claims 1 or 2 or the at least one isolated single domain antibody for use according to any of claims 3 to 8, wherein said at least one isolated single domain antibody specific for the deNEDDylating enzyme NEDP1 is chosen from AbR2DP80, AbR2DP67, AbR2DP34, AbR2DP78, AbR2DP57, AbR2DP12, AbR2PD46 and AbR2DP47.

10. The kit according to claim 9 or the at least one isolated single domain antibody for use according to claim 9, wherein said at least one isolated single domain antibody specific for the deNEDDylating enzyme NEDP1 is chosen from AbR2DP12, AbR2PD46 and AbR2DP47.

11. The kit according to claim 9 or the at least one isolated single domain antibody for use according to claim 9, wherein said at least one isolated single domain antibody specific for the deNEDDylating enzyme NEDP1 is chosen from AbR2DP80, AbR2DP67, AbR2DP34, AbR2DP78, AbR2DP57.
